# EUROPEAN PATENT SPECIFICATION

(11) **EP 3 762 050 B1**
(45) Date of publication and mention of the grant of the patent: **03.12.2025**
(21) Application number: 19712448.0
(22) Date of filing: 07.03.2019
(51) Int. Cl.: A61L 27/26, A61L 27/38, A61L 27/48, A61L 27/56, B33Y 80/00, B33Y 70/00

(54) **NANOCELLULOSE-CONTAINING BIOINKS FOR 3D BIOPRINTING, METHODS OF MAKING AND USING THE SAME, AND 3D BIOSTRUCTURES OBTAINED THEREFROM**
NANOZELLULOSEHALTIGE BIOTINTEN FÜR DAS 3D-BIOPRINTING, VERFAHREN ZU IHRER HERSTELLUNG UND VERWENDUNG UND DARAUS HERGESTELLTE 3D-BIOSTRUKTUREN
ENCRES BIOLOGIQUES CONTENANT DE LA NANOCELLULOSE POUR LA BIO-IMPRESSION 3D, LEURS PROCÉDÉS DE FABRICATION ET D'UTILISATION, ET BIOSTRUCTURES 3D OBTENUES À PARTIR DE CELLES-CI

(30) Priority: 07.03.2018 US 201862639538 P
(43) Date of publication of application: 13.01.2021
(73) Proprietor: GranBio Intellectual Property Holdings, LLC, Atlanta, GA 30326 (US); Regeninx Limited, Tawe Business Village Swansea SA7 9LA (GB)
(72) Inventor: NELSON, Kimberly, Atlanta, Georgia 30317 (US); WHITAKER, Iain, Swansea West Glamorgan SA34SW (GB); JESSOP, Zita, Cardiff West Glamorgan CF119BW (GB); AL-SABAH, Ayesha, Cardiff West Glamorgan CF10 3AL (GB)
(74) Representative: Smart, Jessica Ann
(86) International application number: PCT/US2019/021225
(87) International publication number: WO 2019/173637

(56) References cited:
- WO-A1-2016/100856
- NELSON KIM: "Innovative nanocellulose process breaks the cost barrier", TAPPI JOURNAL, vol. 13, no. 5, 2014, 2014, pages 19 - 23, XP93003298
- DUONG NGUYEN ET AL: "Cartilage Tissue Engineering by the 3D Bioprinting of iPS Cells in a Nanocellulose/Alginate Bioink", SCIENTIFIC REPORTS, vol. 7, no. 1, 1 April 2017 (2017-04-01), XP055393104, DOI: 10.1038/s41598-017-00690-y
- HÉCTOR MARTÍNEZ ÁVILA ET AL: "3D bioprinting of human chondrocyte-laden nanocellulose hydrogels for patient-specific auricular cartilage regeneration", BIOPRINTING, vol. 1-2, 1 March 2016 (2016-03-01), pages 22 - 35, XP055574660, ISSN: 2405-8866, DOI: 10.1016/j.bprint.2016.08.003
- KAJSA MARKSTEDT ET AL: "3D Bioprinting Human Chondrocytes with Nanocellulose-Alginate Bioink for Cartilage Tissue Engineering Applications", BIOMACROMOLECULES, vol. 16, no. 5, 11 May 2015 (2015-05-11), US, pages 1489 - 1496, XP055254675, ISSN: 1525-7797, DOI: 10.1021/acs.biomac.5b00188
- JENNI LEPPINIEMI ET AL: "3D-Printable Bioactivated Nanocellulose-Alginate Hydrogels", ACS APPLIED MATERIALS & INTERFACES, vol. 9, no. 26, 5 July 2017 (2017-07-05), US, pages 21959 - 21970, XP055501600, ISSN: 1944-8244, DOI: 10.1021/acsami.7b02756
- AL-SABAH AYESHA ET AL: "Structural and mechanical characterization of crosslinked and sterilised nanocellulose-based hydrogels for cartilage tissue engineering", CARBOHYDRATE POLYMERS, vol. 212, 18 February 2019 (2019-02-18), pages 242 - 251, XP085615362, ISSN: 0144-8617, DOI: 10.1016/J.CARBPOL.2019.02.057
- JESSOP ET AL.: "Printability of pulp derived crystal, fibril and blend nanocellulose-alginate bioinks for extrusion 3D bioprinting", BIOFABRICATION, 11 February 2019 (2019-02-11), XP002792190, Retrieved from the Internet <URL:https://iopscience.iop.org/article/10.1088/1758-5090/ab0631/pdf> DOI: 10.1088/1758-5090/ab0631

## Description

### PRIORITY DATA

This international patent application claims priority to U.S. Provisional Patent App. No. 62/639,538, filed on March 7, 2018.

### FIELD

The present invention generally relates to bioink compositions for 3D bioprinting.

### BACKGROUND

Three-dimensional (3D) printing, or additive manufacturing, is a process by which an object is created in its three-dimensional form using a specialized printer. The printer receives instructions from a design file that is created in a computer with the help of a 3D modeling program. The file or the digital blueprint of the object to be printed, is then sliced into two-dimensional (2D) representations that are sent to the printer. The layers of material are built according to the information contained in the file; the layers keep on adding until the full object is printed. The process of 3D printing requires much more time and involves significant capital investments as compared to 2D printing, but offers a wide array of advantages, such as the ability, in principle, to print any geometry.

There is now strong interest in industry and academia to construct tissues and organs using 3D-printing technology. The process of printing living cells, tissues, organs and biocompatible scaffolds using 3D printing technologies is called "3D bioprinting". Bioprinting human tissues and organs requires bioink, which includes biological materials such as agarose, alginate, chitosan, collagen, extracellular matrix (ECM), gelatin, fibrin, and hyaluronic acid.

3D printing of biological materials offers numerous advantages. These applications are mainly in the area of drug testing, tissue engineering, and organ transplantation, but other biological applications may emerge in the future.

3D bioprinting is much more complex than conventional 3D printing of polymers or metals, since 3D bioprinting involves functional living biomaterials. Due to challenges posed by the varying sensitivities of living cells and tissues, the appropriate choice of material, cell types, and growth and differentiation factors used, usually determine the structural integrity of the bioprinted tissue.

Since extrusion and inkjet-based technologies have been adapted from conventional 3D printing processes, there are several compatibility and stability issues associated with using these systems with delicate biomaterials. Bioprinted products developed using the aforementioned technologies lack structural integrity at the microscopic level. It is worth noting that the cellular structure at multiple length scales impacts the functionality of the overall artificial tissue; this is crucial for the generation of a fully functional tissue. It is desired for bioinks to simultaneously replicate native tissue microarchitectures and macroarchitectures, overcoming the problems of repeatability and scalability of conventional tissue engineering strategies.

Another common observation related to 3D bioprinting is the fact that the constructed 3D biostructures are largely unstable and have been reported to undergo distortions in shape over time.

Of the three-main 3D bioprinting technologies: extrusion, inkjet and laser-assisted, extrusion is the most versatile, fast, scalable, and cost-effective, and therefore is the predominant technique today. Extrusion bioprinting relies on extruding bioinks with suitable mechanical properties (viscosity, elasticity, and shear-thinning) through a nozzle using either mechanical (piston or screw-driven) or pneumatic forces. Extrusion-based bioprinting (e.g. bioplotting or fused-deposition modeling) involves dispensing viscous bioink through a nozzle or syringe. After printing, the constructs can be solidified (e.g., gelled) either physically or chemically layer by layer. One of the main drawbacks of extrusion-based bioprinting is its reliance on optimal material viscosity, which when not achieved, can lead to leaks and affect the resolution of the final tissue construct, limiting the complexity of the biostructures (especially those with overhang). Suitable bioinks must also exhibit cellular viability under shear and/or high temperature, adhesion, proliferation, and differentiation. Also, suitable bioinks should be capable of producing biostructures with acceptable mechanical stiffness.

Although it is easier to tailor the biomechanical properties of synthetic-polymer bioinks such as polyacrylamides and polyethylene glycols to suit extrusion techniques, the biocompatibility and tissue regenerative potential of these polymers are inferior to natural, non-synthetic bioinks such as gelatin, agarose, alginate, hyaluronic acid, and collagen, which mimic the natural extracellular matrix environment.

One of the main challenges for extrusion 3D bioprinting is therefore the identification of non-synthetic bioinks with suitable rheological properties as well as biocompatibility. Many polymers offer suitable rheological properties but are not biocompatible. On the other hand, many biological materials are biocompatible but do not confer suitable rheological properties to the bioink, to make it useful for bioprinting.

Ensuring high resolution or fidelity of bioprinted constructs and finding the optimal bioink remain major hurdles for printing complex biological structures. Higher resolutions not only allow better replication of native architecture but can also control pore size and interconnectivity, which is important when considering that diffusion distances of over 400-500 mm may limit oxygen transport and hence cell viability. Currently stereolithography, and inkjet-based techniques provide good resolution but are limited by the lack of appropriate biomaterials, lower cell viabilities, and poor mechanical strength. Laser-assisted bioprinters can print at a microscale resolution, but preparation of individual ribbons for deposition can be time-consuming and not cost-effective.

For additional background information, see Jessop et al., "3D bioprinting for reconstructive surgery: Principles, applications and challenges", Journal of Plastic, Reconstructive & Aesthetic Surgery (2017) 70, 1155-1170.

Tissue engineering promises the creation of solid organ transplants that would save thousands of lives each year and lift a chronic health burden. In addition, tissue engineering holds the potential to restore form and function to patients born with congenital deformity or who acquire it later in life due to trauma or malignancy.

Cartilage is an avascular tissue containing chondrocytes with limited self-regenerative properties. There is a demand for replacing damaged cartilage tissue with alternative approaches, such as tissue engineering, since the cartilage will not repair itself. Reconstruction of facial cartilage defects, from trauma, burns, skin cancer, or congenital conditions, currently relies on using autologous grafts, most commonly from the costochondral site, with significant donor site morbidity. Tissue-engineered cartilage constructs, based on non-specific cell seeding of scaffolds, fail to replicate native tissue anisotropy and are therefore mechanically unstable and prone to degradation, calcification, and inflammation *in vivo,* especially when synthetic biomaterials are used.

Contemporary research into 3D bioprinting of cartilage has identified several potential natural bioink materials, including fibrin, alginate, gelatin, gelatinous protein mixtures secreted by Engelbreth-Holm-Swarm (EHS) mouse sarcoma cells, and nanocellulose, which have been used as scaffolds for facilitating cell homing from neighboring healthy tissue or deposition of extracellular matrix following the addition of cellular components such as chondrocytes or mesenchymal stem cells. However, the application of the more commonly used bioinks can be a challenge due to suboptimal printability. Pure alginate formulations, in particular, have been identified as providing poor post-printing shape fidelity even when viscosity is increased.

Nanocellulose, composed of parallel linear polysaccharide molecules and extracted from the biosynthesis of plants or bacteria, is an emerging class of advanced naturally derived nanomaterial. It is promising due to its attractive physicochemical properties, extraordinarily high stiffness (100-200 GPa) and strength, alongside its abundance and sustainability. Bacterial nanocellulose has shown promise for tissue engineering applications due to its biocompatibility, nanostructure, functionalization potential, water-holding capacity, and similarities in morphology to collagen, thereby providing cell support. The unique biomechanical and rheological properties due to the high aspect ratio of bacterial cellulose have also meant that it has potential utility as a bioink for 3D bioprinting. However, the current techniques for producing bacterial nanocellulose are limited by the huge substrate costs of supporting bacterial growth, low yield of products, scalability, and concerns regarding residual bacterial toxins/epitopes, which has precluded widespread use. Nanocellulose can also be generated from biomass using expensive enzymatic treatments or using concentrated acids with poor chemical recovery, making clinical translation and scale-up problematic.

Hydrogels have been pointed out as attractive materials for bioinks because in general hydrogels are biocompatible, show low cytotoxicity, and have high water content, similar to extracellular matrix. Common hydrogels evaluated for 3D bioprinting are the natural polymers collagen, hyaluronic acid, chitosan, and alginate. To be suitable for 3D bioprinting, a hydrogel must be viscous enough to keep its shape during printing and must be capable of crosslinking to retain the 3D structure after bioprinting. Crosslinking can occur by temperature change, UV photopolymerization, and/or ionic crosslinking. For example, alginates isolated from brown algae have the ability to crosslink with the addition of divalent cations. Pure-alginate bioinks have proven to be inferior due to thermosensitive viscosity and uncontrollable printability. Although the viscosity of alginate can be increased by varying the concentration and molecular weight, it has not been sufficient for achieving shape fidelity while printing.

Despite wide interest in 3D bioprinting research, a truly universal bioink does not heretofore exist. A universal bioink would address many of the known problems, including thermosensitivity, swelling or shrinkage post-printing in culture media, lack of shear-thinning behavior, slow gelation, poor resolution, poor post-printing shape fidelity, cell adhesion, bioactivity and cell survival, cell differentiation into tissue, and a range of topographies to provide an optimal environment for cell adhesion, migration, proliferation, and differentiation. What is desired is a bioink composition that is printable, is capable of maintaining complex macrostructures, and provides an environment in which living cells can thrive and differentiate. Methods of making and using such a bioink are also sought.

WO 2016/100856 A1 in accordance with its abstract states "The present invention relates to biomaterial in the form of dispersion of cellulose nanofibrils with extraordinary shear thinning properties which can be converted into desire 3D shape using 3D Bioprinting technology. In this invention cellulose nanofibril dispersion, is processed through different mechanical, enzymatic and chemical steps to yield dispersion with desired morphological and rheological properties to be used as bioink in 3D Bioprinter. The processes are followed by purification, adjusting of osmolarity of the material and sterilization to yield biomaterial which has cytocompatibility and can be combined with living cells. Cellulose nanofibrils can be produced by microbial process but can also be isolated from plant secondary or primary cell wall, animals such as tunicates, algae and fungi. The present invention describes applications of this novel cellulose nanofibrillar bioink for 3D Bioprinting of tissue and organs with desired architecture".

### SUMMARY

The presently claimed invention provides a bioink composition for 3D bioprinting, the bioink composition comprising:
(a) nanocellulose in the form of a combination of nanocellulose crystals and nanocellulose fibrils;
(b) alginate in the form of alginic acid and/or an alginate salt; and
(c) water,
wherein the alginate is ionically crosslinkable in the presence of an ionic crosslinking agent.

In some embodiments, the nanocellulose is present at a nanocellulose concentration from 1% (w/v) to 10% (w/v). In certain embodiments, the nanocellulose is present at a nanocellulose concentration from 3% (w/v) to 6% (w/v).

In some embodiments, the alginate is present at an alginate concentration from 0.1% (w/v) to 5% (w/v).

Preferably, the nanocellulose is hydrophilic nanocellulose that contains little or no lignin.

In some embodiments of the invention, the bioink composition is a cell-free bioink. In other embodiments, the bioink composition is a cell-laden bioink containing viable human cells (or other animal cells). In certain embodiments, the viable human cells are human nasoseptal chondrocytes. The viable human cells may be present in the bioink composition at a cell concentration from 10⁵ to 10⁷ cells per milliliter of the bioink composition, such as 2 × 10⁶ cells/mL, for example.

The nanocellulose is in the form of a combination (blend) of nanocellulose crystals and nanocellulose fibrils.

In the blend of nanocellulose crystals and nanocellulose fibrils, the nanocellulose may be characterized by a thermal-decomposition onset temperature of at least 250°C, 260°C, 270°C, 280°C, 290°C, or 300°C.

The nanocellulose preferably contains less than 0.05 wt% sulfur, such as about 0.02 wt% sulfur or less. Preferably, the nanocellulose contains no sulfate half-ester groups attached to the surface of the nanocellulose particles.

In preferred embodiments, the nanocellulose is derived from fractionation of lignocellulosic biomass in the presence of sulfur dioxide and ethanol (and water) to generate cellulose, hemicellulose, and lignin, followed by mechanical refining of the cellulose to generate nanocellulose.

Preferably, the nanocellulose is not bacterial nanocellulose, is not derived from tunicates, and is not obtained via enzymatic hydrolysis of lignocellulosic biomass or cellulose.

The nanocellulose preferably has a zeta potential of at least -10 mV or more negative, such as at least -20 mV or more negative.

The nanocellulose is preferably shear-thinning so that the bioink can be conveniently 3D-printed via extrusion.

The bioink composition may further comprise an ionic crosslinking agent, which is typically added at a later time, when crosslinking is desired. In some embodiments, the ionic crosslinking agent includes a divalent cation, such as (but not limited to) a divalent metal cation selected from the group consisting of Ca²⁺, Sr²⁺, Ba²⁺, and combinations thereof. An exemplary ionic crosslinking agent is calcium chloride, CaCl₂.

In various embodiments, the bioink composition further comprises one or more bioink additives selected from the group consisting of growth promoters, growth factors, vitamins, minerals, enzymes, proteins, sugars, sugar alcohols, acids, bases, salts, buffers, stabilizers, dissolved oxygen, and antibiotics.

The disclosed bioink composition is non-cytotoxic to the human cells selected for bioprinting.

Other variations of the invention that are disclosed but not presently claimed provide a method of making a bioink composition for 3D bioprinting, the method comprising:
(a) obtaining nanocellulose in the form of nanocellulose crystals, nanocellulose fibrils, or a combination thereof, wherein the nanocellulose crystals (if present) are characterized by a nanocrystal thermal-decomposition onset temperature of at least 290°C, and wherein the nanocellulose fibrils (if present) are characterized by a nanofibril thermal-decomposition onset temperature of at least 240°C;
(b) combining the nanocellulose with alginate in an aqueous solution, wherein the alginate is in the form of alginic acid and/or an alginate salt, and wherein the alginate is ionically crosslinkable in the presence of an ionic crosslinking agent;
(c) sterilizing the aqueous solution;
(d) optionally adding a plurality of viable human cells to the aqueous solution;
   and
(e) recovering the aqueous solution as a bioink composition for 3D bioprinting.

In some embodiments, the nanocellulose is present in the bioink composition at a nanocellulose concentration from 1% (w/v) to 10% (w/v). In these or other embodiments, the alginate is present in the bioink composition at an alginate concentration from 0.1% (w/v) to 5% (w/v).

Preferably, in step (a), the nanocellulose is derived from fractionation of lignocellulosic biomass in the presence of sulfur dioxide and ethanol (and water) to generate cellulose, hemicellulose, and lignin, followed by mechanical refining of the cellulose to generate the nanocellulose.

In some preferred embodiments, the nanocellulose is in the form of a combination (blend) of nanocellulose crystals and nanocellulose fibrils.

In some embodiments, in step (c), sterilizing utilizes a technique selected from the group consisting of steam sterilization, UV sterilization, ethanol sterilization, and combinations thereof. When steam sterilization is used, step (c) separately sterilizes the nanocellulose (or a liquid solution containing the nanocellulose) and the alginate (or a liquid solution containing the alginate) prior to preparing the aqueous solution, and preferably the alginate is not sterilized by steam sterilization since alginate is thermally sensitive. Alginate is preferably UV-sterilized and/or ethanol-sterilized, rather than steam-sterilized.

The produced bioink composition may be a cell-free bioink or a cell-laden bioink. In some embodiments, the bioink composition is a cell-laden bioink containing viable human cells, such as (but not limited to) human nasoseptal chondrocytes. Viable human cells may be present in the bioink composition at a cell concentration from 10⁵ to 10⁷ cells per milliliter of the bioink composition, for example.

Other variations that are disclosed but not presently claimed provide a method of making a 3D-printed biostructure, the method comprising:
(a) obtaining nanocellulose in the form of nanocellulose crystals, nanocellulose fibrils, or a combination thereof, wherein the nanocellulose crystals (if present) are characterized by a nanocrystal thermal-decomposition onset temperature of at least 290°C, and wherein the nanocellulose fibrils (if present) are characterized by a nanofibril thermal-decomposition onset temperature of at least 240°C;
(b) combining the nanocellulose with alginate in an aqueous solution, wherein the alginate is in the form of alginic acid and/or an alginate salt, and wherein the alginate is ionically crosslinkable in the presence of an ionic crosslinking agent;
(c) sterilizing the aqueous solution;
(d) adding a plurality of viable human cells to the aqueous solution, to form a cell-laden bioink;
(e) depositing multiple layers of the cell-laden bioink onto a substrate or surface;
(f) exposing the multiple layers of the cell-laden bioink to an ionic crosslinking agent, during step (e) and/or following step (e), to ionically crosslink the alginate; and
(g) recovering a 3D-printed biostructure containing the multiple layers of the cell-laden bioink.

In some embodiments, the nanocellulose is present in the cell-laden bioink at a nanocellulose concentration from 1% (w/v) to 10% (w/v). In these or other embodiments, the alginate is present in the cell-laden bioink at an alginate concentration from 0.1% (w/v) to 5% (w/v).

Preferably, the nanocellulose is derived from fractionation of lignocellulosic biomass in the presence of sulfur dioxide and ethanol (and water) to generate cellulose, hemicellulose, and lignin, followed by mechanical refining of the cellulose to generate the nanocellulose.

In some preferred embodiments, the nanocellulose is in the form of a combination (blend) of nanocellulose crystals and nanocellulose fibrils.

Step (c) may utilize a sterilizing technique selected from the group consisting of steam sterilization, UV sterilization, ethanol sterilization, and combinations thereof. When steam sterilization is used, step (c) separately sterilizes the nanocellulose (or a liquid solution containing the nanocellulose) and the alginate (or a liquid solution containing the alginate) prior to preparing the aqueous solution, and preferably the alginate is not sterilized by steam sterilization since alginate is thermally sensitive. Alginate is preferably UV-sterilized and/or ethanol-sterilized, rather than steam-sterilized.

In some embodiments, the viable human cells are human nasoseptal chondrocytes, and the 3D-printed biostructure contains human cartilage.

In some embodiments, depositing in step (e) utilizes extrusion (i.e., extrusion-based 3D bioprinting).

In some embodiments, in step (f), crosslinking is performed between formation of each of the multiple layers of the cell-laden bioink. In these or other embodiments, in step (f), crosslinking is performed after formation of all of the multiple layers of the cell-laden bioink.

The ionic crosslinking agent may include a divalent cation, such as Ca²⁺, Sr²⁺, Ba²⁺, or a combination thereof.

In some embodiments, step (f) utilizes an aerosol of the ionic crosslinking agent, wherein the aerosol is continuously or periodically exposed to the multiple layers of the cell-laden bioink.

The 3D-printed biostructure, disclosed but not presently claimed, may be characterized by a porous 3D network of interconnecting compact nanocellulose particles, intimately dispersed within the alginate that is ionically crosslinked. Preferably, the nanocellulose within the 3D-printed biostructure contains a range of pore sizes, such as from about 50 nanometers to about 10 microns. In some embodiments, the nanocellulose within the 3D-printed biostructure contains pore sizes as small as 55 nanometers and as large as 12 microns.

The final 3D-printed biostructure may be selected from the group consisting of an ear, a nose, a cartilaginous joint, a trachea ring, a larynx, a costal cartilage bar, or a portion thereof. The 3D-printed biostructure may be an arbitrary geometric shape.

### BRIEF DESCRIPTION OF THE FIGURES

FIG. 1 summarizes a qualitative analysis of various alginate concentrations assessed against ease of dissolution, flow, mixing with nanocellulose blend (NCB) at room temperature, and uncrosslinked post-printing construct stability, in the Examples.
FIG. 2 summarizes an analysis of alginate crosslinking time (minutes) and crosslinking strength, using aerosolized CaCl₂ for crosslinking, in the Examples.
FIG. 3 shows the results of a grid thickness assay to test bioprinting resolution for nanocellulose-containing bioinks, in the Examples.
FIG. 4 displays G-code in graphical format of the shapes used for complex structure testing in the Examples; inset A is a hollow cylinder, inset B is a cylinder, inset C is nasal cartilage, inset D is a cube, inset E is a right ear, and inset F is a 4-sided pyramid.
FIG. 5 summarizes alginate crosslinking optimization with different CaCl₂ concentrations, in the Examples.
FIG. 6 shows TEM images of nanocellulose-alginate bioinks before and after crosslinking, in the Examples.
FIG. 7 provides a comparison of cylindrical construct volume bioprinted using three different bioink formulations (CNC-AG, NCB-AG, and CNF-AG) crosslinked with either 0.1 M or 0.5 M CalCl₂, in the Examples.
FIG. 8 shows the change in construct (3D biostructure) volume relative to time 0 at 24 and 72 hours, in the Examples.
FIG. 9 reveals geometric structures printed using NCB-AG bioink for complex shape testing, in the Examples.
FIG. 10 shows rheological properties of bioinks containing alginate but not nanocellulose, in the Examples.
FIG. 11 shows viscosity of pure-alginate bioinks as a function of shear rate, in the Examples.
FIG. 12 shows rheological properties of nanocellulose-alginate bioinks at 1 Hz, in the Examples.
FIG. 13 shows the viscosity for different nanocellulose-alginate bioinks as a function of shear rate, for CNC, NCB, and CNF bioinks combined with 1.25% alginate (w/v), in the Examples. The x-axis symbol "-s" denotes units of inverse seconds, s⁻¹ (Hz).
FIG. 14 shows the viscosity for different nanocellulose-alginate bioinks as a function of shear rate, for CNC, NCB, and CNF bioinks combined with 2.5% alginate (w/v), in the Examples. The x-axis symbol "-s" denotes units of inverse seconds, s⁻¹ (Hz).
FIG. 15 shows the viscosity for different nanocellulose-alginate bioinks as a function of shear rate, for CNC, NCB, and CNF bioinks combined with 5% alginate (w/v), in the Examples. The x-axis symbol "-s" denotes units of inverse seconds, s⁻¹ (Hz).
FIG. 16 displays viability of human nasoseptal chondrocytes after bioprinting, for (A) nanocellulose crystal (CNC-AG), (B) nanocellulose blend (NCB-AG), and (C) nanocellulose fibril (CNF-AG) bioink formulations, in the Examples.
FIG. 17 shows human nasoseptal chondrocyte viability in crystal/fibril blended nanocellulose bioink, in 3D cell culture, versus cells only (2D cell culture), in the Examples.
FIG. 18 summarizes the results of a lactate dehydrogenase (LDH) cytotoxicity assay, where cytotoxicity of human nasoseptal chondrocytes in 2D culture is compared to cytotoxicity of human nasoseptal chondrocytes post-bioprinting with NCB-AG bioink, in the Examples.
FIG. 19 shows metabolic activity of human nasoseptal chondrocytes in NCB-AG bioink at 4, 12, and 24 hours, in the Examples.
FIG. 20 displays scanning electron microscopy (SEM) images of alginate bioink and nanocellulose-containing bioink constructs following bioprinting, in the Examples.
FIG. 21 presents a stress-strain curve for bioprinted constructs, in the Examples.
FIG. 22 shows quantitative reverse transcription polymerase chain reaction (qRT-PCR) analysis of relative gene expression of nasoseptal populations cultured in alginate-only bioinks versus nanocellulose-containing bioinks, in the Examples.
FIG. 23 shows SEM images (scale bars of 10, 20, and 50 µm from bottom to top) of nanocellulose-containing bioink within 24 hours of being mixed with nasoseptal chondrocytes (left panels) and after two weeks in culture with nasoseptal chondrocytes (right panels), in the Examples.
FIG. 24 presents a histological analysis of 3D-bioprinted cartilage constructs using cells derived from nasoseptal cartilage, in the Examples.

### DETAILED DESCRIPTION OF SOME EMBODIMENTS

The presently claimed invention is defined by the appended claims. The more general description of the invention is provided for illustrative purposes. Embodiments not falling under these claims are for reference purpose only.

As used in this specification and the appended claims, the singular forms "a," "an," and "the" include plural referents unless the context clearly indicates otherwise. Unless defined otherwise, all technical and scientific terms used herein have the same meaning as is commonly understood by one of ordinary skill in the art to which this invention belongs. All composition numbers and ranges based on percentages are weight percentages, unless indicated otherwise. All ranges of numbers or conditions are meant to encompass any specific value contained within the range, rounded to any suitable decimal point.

Unless otherwise indicated, all numbers expressing parameters, reaction conditions, concentrations of components, and so forth used in the specification and claims are to be understood as being modified in all instances by the term "about." Accordingly, unless indicated to the contrary, the numerical parameters set forth in the following specification and attached claims are approximations that may vary depending at least upon a specific analytical technique.

The term "comprising," which is synonymous with "including," "containing," or "characterized by" is inclusive or open-ended and does not exclude additional, unrecited elements or method steps. "Comprising" is a term of art used in claim language which means that the named claim elements are essential, but other claim elements may be added and still form a construct within the scope of the claim.

As used herein, the phrase "consisting of" excludes any element, step, or ingredient not specified in the claim. When the phrase "consists of" (or variations thereof) appears in a clause of the body of a claim, rather than immediately following the preamble, it limits only the element set forth in that clause; other elements are not excluded from the claim as a whole. As used herein, the phrase "consisting essentially of" limits the scope of a claim to the specified elements or method steps, plus those that do not materially affect the basis and novel characteristic(s) of the claimed subject matter.

With respect to the terms "comprising," "consisting of," and "consisting essentially of," where one of these three terms is used herein, the presently disclosed and claimed subject matter may include the use of either of the other two terms. Thus in some embodiments not otherwise explicitly recited, any instance of "comprising" may be replaced by "consisting of" or, alternatively, by "consisting essentially of."

Some variations of the present invention are predicated on unique bioink compositions that have beneficial rheological properties for extrusion-based 3D bioprinting. The disclosed bioink compositions contain nanocellulose with a wide variation in pore sizes and a tunable topography, mimicking the natural extracellular matrix found in tissues and organs. The disclosed bioink compositions are universally applicable to a variety of cell types.

The invention provides a bioink composition for 3D bioprinting, the bioink composition comprising:
(a) nanocellulose in the form of a combination of nanocellulose crystals and nanocellulose fibrils;
(b) alginate in the form of alginic acid and/or an alginate salt; and
(c) water,
wherein the alginate is ionically crosslinkable in the presence of an ionic crosslinking agent.

In some embodiments, the nanocellulose is present in the bioink composition at a nanocellulose concentration from 1% (w/v) to 10% (w/v), such as 1.5%, 2%, 2.5%, 3%, 3.5%, 4%, 4.5%, 5%, 5.5%, 6%, 6.5%, 7%, 7.5%, 8%, 8.5%, 9%, or 9.5% (all w/v). In certain embodiments, the nanocellulose is present at a nanocellulose concentration from 3% (w/v) to 6% (w/v). Note that other nanocellulose concentrations may be used, including lower than 1% (w/v) or higher than 10% (w/v), depending on factors such as nanocellulose molecular weight, nanocellulose particle size, concentrations of other components in the bioink, intended biostructure, etc.

Alginate is a linear copolymer of β-D-mannuronic acid and α-L-guluronic acid. In some embodiments, the alginate is present in the bioink composition at an alginate concentration from 0.1% (w/v) to 5% (w/v), such as 0.2%, 0.3%, 0.4%, 0.5%, 0.6%, 0.7%, 0.8%, 0.9%, 1%, 1.5%, 2%, 2.5%, 3%, 3.5%, 4%, or 4.5% (all w/v). Note that other alginate concentrations may be used, including lower than 0.1% (w/v) or higher than 5% (w/v), depending on factors such as alginate molecular weight, concentrations of other components in the bioink, intended degree of crosslinking in the biostructure, etc.

In some embodiments, the nanocellulose and alginate are collectively present in the bioink composition at a concentration from 1% (w/v) to 15% (w/v), such as 0.2%, 0.3%, 0.4%, 0.5%, 0.6%, 0.7%, 0.8%, 0.9%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 10.5%, 11%, 11.5%, 12%, 12.5%, 13%, 13.5%, 14%, or 14.5% (all w/v). In some embodiments, the nanocellulose and alginate are collectively present in the bioink composition at least at 4% (w/v), at least at 5% (w/v), or at least at 6% (w/v).

The weight ratio of nanocellulose to alginate in the bioink composition may vary, such as from 1 to 40, e.g. 1.5, 2, 2.5, 3, 3.5, 4, 4.5, 5, 6, 7, 8, 9, 10, 15, 20, 25, 30, or 35. In some preferred embodiments, the weight ratio of nanocellulose to alginate in the bioink composition is about 4 (i.e., from 3.6 to 4.4) or about 5 (i.e., from 4.6 to 5.4).

Besides nanocellulose and alginate, water is present in the bioink composition. When there are no other components other than nanocellulose, alginate, and water, then water is the balance (e.g., 3% nanocellulose, 0.75% alginate, and 100 - 3.75 = 96.25% water, all on a w/v basis). When additives are present in the bioink composition, then the water content will be less than 100 minus the sum of nanocellulose and alginate concentrations. Note that although the bioink composition may initially be provided as a dry-powder precursor, water is a necessary component for bioprinting (due to water requirements for cell maintenance and development). Normally, the bioink composition explicitly includes water. It will be understood by a person skilled in the bioprinting art that there are implied instructions to add water to a dry-powder precursor (of nanocellulose + alginate) to form the bioink composition, which therefore can be regarded as including water in the first place.

In some embodiments of the invention, the bioink composition is a cell-free bioink. In other embodiments, the bioink composition is a cell-laden bioink containing viable human cells (or other animal cells). In certain embodiments, the viable human cells are human nasoseptal chondrocytes. The viable human cells may be present in the bioink composition at a cell concentration from 10⁵ to 10⁷ cells per milliliter of the bioink composition, for example, such as 2 × 10⁶ cells/mL as one exemplary embodiment.

The nanocellulose is in the form of a combination (blend) of nanocellulose crystals and nanocellulose fibrils. As will be explained in more detail below, there are benefits (observed experimentally; see Examples) when a blend of nanocellulose crystals and nanocellulose fibrils is utilized in the bioink.

A blend of nanocellulose crystals and fibrils may contain from 1% to 99% nanocellulose crystals and from 99% to 1% of nanocellulose fibrils, respectively. In various embodiments, the blend of nanocellulose crystals and fibrils contains 2%, 5%, 10%, 15%, 20%, 25%, 30%, 35%, 40%, 45%, 50%, 55%, 60%, 65%, 70%, 75%, 80%, 85%, 90%, 95%, or 98% (all weight percentages) of nanocellulose crystals, with the rest of the nanocellulose being nanocellulose fibrils.

One characteristic of a nanocellulose blend is the wide range of particles sizes present, since nanocellulose fibrils are much larger than nanocellulose crystals. The nanocellulose crystal width may vary from about 2 nanometers to about 10 nanometers, or from about 3 nanometers to about 6 nanometers, for example. The nanocellulose crystal length may vary from about 50 nanometers to about 500 nanometers, or from about 100 nanometers to about 350 nanometers, for example. The nanocellulose fibril width may vary from about 5 nanometers to about 100 nanometers, or from about 10 nanometers to about 50 nanometers, for example. The nanocellulose fibril length may vary from about 200 nanometers to about 10 microns, or from about 400 nanometers to about 3 microns, for example. The average nanocellulose particle width in the blend may vary from about 3 nanometers to about 50 nanometers, such as from about 5 nanometers to about 30 nanometers. The average nanocellulose particle length in the blend may vary from about 50 nanometers to about 5 microns, such as from about 100 nanometers to about 2 microns.

Preferably, the nanocellulose is hydrophilic nanocellulose that contains little or no lignin. Lignin-coated nanocellulose is known to be at least partially hydrophobic, which is normally not desirable for 3D bioprinting. Therefore, in preferred embodiments, the nanocellulose contains no greater than 0.5 wt% lignin, preferably no greater than 0.1 wt%, more preferably no greater than 0.01 wt% lignin, and most preferably no detectible lignin. These low levels of lignin (if any) cause the nanocellulose to be hydrophilic and give higher viscosity and water-holding capacity, which is preferred.

The "thermal-decomposition onset temperature" of the nanocellulose is defined by a thermal gravimetric analysis of the nanocellulose. Thermal gravimetric analysis, also known as thermogravimetric analysis (TGA), is a technique in which the mass of a substance is monitored as a function of temperature or time as the sample specimen is subjected to a controlled temperature program in a controlled atmosphere. The thermal gravimetric analysis may utilize a PerkinElmer STA6000 Simultaneous Thermal Analyzer (London, UK). The abscissa (x-axis) is temperature and the ordinate (y-axis) is weight percent (%). A descending TGA thermal curve indicates a weight loss occurred. From the TGA thermal curve, the extrapolated onset temperature can be calculated, denoting the temperature at which the weight loss begins. The extrapolated onset temperature is a reproducible temperature calculation and it is specified to be used by ASTM and ISO for TGA. The extrapolated onset is the point of intersection of the tangent drawn at the point of greatest slope on the leading edge of the peak with the extrapolated base line. Another calculation that is useful is the first derivative of the weight loss curve. The first-derivative peak temperature indicates the point of greatest rate of change on the weight-loss curve. This is also known as the inflection point.

For purposes of this specification, to determine the thermal-decomposition onset temperature, nanocellulose samples are heated from 50°C to 600°C at a heating rate of 10°C/min under nitrogen atmosphere with a flow rate of 40 mL/min, using a PerkinElmer STA6000 Simultaneous Thermal Analyzer. See Kyle et al., "Characterization of pulp derived nanocellulose hydrogels using AVAP® technology", Carbohydrate Polymers 198 (2018) 270-280. The nanocellulose mass is measured as a function of temperature. Nanocellulose mass loss, other than water loss, indicates nanocellulose thermal decomposition. The extrapolated onset temperature (see above) from the TGA graph is the estimated thermal-decomposition onset temperature.

Employing a blend of nanocellulose crystals and nanocellulose fibrils, the nanocellulose is characterized by a thermal-decomposition onset temperature of at least any of 250°C, 260°C, 265°C, 270°C, 280°C, 290°C, or 300°C.

Without being limited by speculation, the nanocellulose thermal-decomposition onset temperature is believed to depend on the starting lignocellulosic feedstock used to make the nanocellulose, as well as the biorefining process conditions to make the nanocellulose. When comparing the nanocellulose of this invention with other processes to make nanocellulose, such as sulfuric acid hydrolysis for nanocrystals or 2,2,6,6-tetramehylpiperidine-1-oxy radical (TEMPO) treatment to make nanofibrils, the same starting feedstock should be used for comparison. In the case of nanocrystals, the nanocellulose crystals provided herein preferably have a higher thermal-decomposition onset temperature compared to sulfuric acid hydrolysis of the same feedstock, and the nanocellulose fibrils provided herein preferably have a higher thermal-decomposition onset temperature compared to TEMPO treatment of the same feedstock.

As noted above, the thermal gravimetric analysis also provides the "thermal-decomposition maximum-rate temperature" which is the temperature at which the rate of thermal decomposition is measured to be the highest, i.e., the first-derivative peak temperature.

Employing a blend of nanocellulose crystals and nanocellulose fibrils, the nanocellulose is characterized by a thermal-decomposition maximum-rate temperature of at least any of 290°C, 300°C, 310°C, 320°C, 330°C, 340°C, 350°C, or 360°C.

The nanocellulose preferably contains less than 0.05 wt% sulfur, such as about 0.02 wt% sulfur or less, including no detectible sulfur. Preferably, the nanocellulose contains no sulfate half-ester groups attached to the surface of the nanocellulose particles, as these groups may impart toxicity to human cells, in addition to reducing the thermal stability of the nanocellulose.

In preferred embodiments, the nanocellulose is derived from fractionation of lignocellulosic biomass in the presence of sulfur dioxide, ethanol, and water to generate cellulose, hemicellulose, and lignin, followed by mechanical refining of the cellulose to generate nanocellulose.

Preferably, the nanocellulose is not bacterial nanocellulose, is not derived from tunicates, and is not obtained via enzymatic hydrolysis of lignocellulosic biomass or cellulose.

The nanocellulose preferably has a zeta potential of at least -10 mV or more negative, or at least -20 mV or more negative (e.g., -15 mV, -16 mV, -17 mV, -18 mV, -19 mV, -20 mV, -21 mV, -22 mV, -23 mV, -24 mV, or -25 mV). Zeta potential measurements (estimated as surface charge) may be carried out using a Zetasizer NanoZS (Malvern Instruments, UK), for example. The zeta potential measures the mobility of charged particles across an electric field. A zeta potential that is at least -20 mV or more negative assists the nanocellulose suspension to form a stable colloid gel.

The nanocellulose is preferably shear-thinning so that the bioink can be conveniently 3D-printed via extrusion. In rheology, shear-thinning is the non-Newtonian behavior of fluids whose viscosity decreases under shear strain.

The bioink composition may further comprise an ionic crosslinking agent, which is typically added at a later time, when alginate crosslinking is desired. In some embodiments, the ionic crosslinking agent includes a divalent cation, such as (but not limited to) a divalent metal cation selected from the group consisting of Ca²⁺, Sr²⁺, Ba²⁺, and combinations thereof. The cation is typically, although not necessarily, present as a neutral salt; for example, Ca²⁺ may be present as calcium chloride, CaCl₂. Other divalent metal cations technically work to crosslink alginate, such as Pb²⁺, Cu²⁺, Cd²⁺, Ni²⁺, Zn²⁺, and Mn²⁺, but these are less preferred due to toxicity. The ionic crosslinking agent may also employ divalent organic cations.

The gelation and crosslinking of the alginate are mainly achieved by the exchange of sodium ions from the guluronic acids with the divalent cations. The divalent cations bind to the α-L-guluronic acid blocks in a highly cooperative manner, and the size of the cooperative unit is typically more than 20 monomers. Each alginate chain can dimerize to form junctions with many other chains, forming gel networks rather than insoluble precipitates.

In various embodiments, the bioink composition further comprises one or more bioink additives selected from the group consisting of growth promoters, growth factors, vitamins, minerals, enzymes, proteins, sugars (e.g., glucose), sugar alcohols (e.g., mannitol), acids, bases, salts (e.g., sodium chloride), buffers, stabilizers, dissolved oxygen, and antibiotics. The amounts of additives, if any, will generally be dictated by the type of cells included in the cell-laden bioink and the intended 3D biostructure. Acids, bases, and buffers may be included to maintain a desired pH for the bioink, such as a pH from about 6 to about 8, or from about 6.5 to about 7.5.

The disclosed bioink compositions are non-cytotoxic to human cells, or at least the selected human cells that are to be bioprinted. Reference is made herein to the Examples for further discussion of cytotoxicity.

The bioink composition may be provided in pre-packaged, pre-sterilized form. In some embodiments, the bioink composition is pre-packaged but not sterilized, accompanied with instructions to sterilize (e.g., in a steam autoclave) at a later time, prior to bioprinting. The pre-packaging may be in small containers, tubes, vials, jars, bags, and the pre-packaging material may be glass, plastic, coated paper, etc. In certain embodiments, the bioink composition is provided in dry form, or at least with reduced water content, accompanied with instructions to add water to the composition prior to bioprinting. In some embodiments, the bioink composition is part of a kit that includes pre-packaged, pre-sterilized bioink along with bioprinting instructions that are tailored for a specific cell type and/or specific biostructure.

The disclosed bioink compositions have beneficial rheology for bioprinting and produce porous fibrillar networks with interconnecting compact high-aspect-ratio nanoparticles and favorable pore sizes for cellular ingress and maintenance. The pore and fiber networks are advantageous for tissue-engineering applications. The reversible stress softening behavior enables shear-thinning during bioprinting (e.g., through a nozzle) while giving post-printing shape fidelity. The bioinks provided herein are printable, capable of maintaining complex macrostructures, and able to provide an environment in which cells can thrive and differentiate.

The rheological properties of these bioink compositions are ideal, in particular, for extrusion bioprinting. The shear-thinning (pseudoelastic) behavior allows flow through a bioprinter nozzle at low shear rates, reducing the mechanical stress exerted on cells. A high zero-shear viscosity maintains shape fidelity following bioprinting and prevents collapse of complex 3D-bioprinted constructs.

The bioink compositions and the 3D biostructures may be characterized by a wide topography range. In particular, variable pore sizes (such as from about 10 nanometers to about 100 microns, or from about 50 nanometers to about 10 microns) favors a wide variety of cell types. The wide range of topographies mimics native collagen fiber sizes in the extracellular matrix to increase bioactive signals to cells. Also, the tunable topography (as a function of type and amount of nanocellulose) provides the ability to customize the bioink for certain cell types.

The bioink compositions may be characterized by beneficial swelling properties. Preferably, the bioink contains suitable concentrations of nanocellulose and ionic crosslinking agent to allow enough swelling for cell nutrition under standard culture conditions, while maintaining post-printing shape fidelity. Once bioprinted into specified 3D biostructures, the resulting biomaterial is resistant to swelling or shrinkage in culture, providing reproducible results.

The bioink compositions are thermoresistant due to the inclusion of thermoresistant nanocellulose, as described above. This property allows autoclave sterilization at 121°C and allows bioprinting at room temperature (rather than colder temperatures) and enables cell culture at biologically optimal temperatures, such as 37°C. The nanocellulose does not disintegrate when under standard culture conditions and at human body temperature.

Note that the disclosed bioink compositions are useful as rheology modifiers for current hydrogels already being used for culture of specific cell types-thereby making previously unprintable hydrogels printable. Disclosed but not part of the invention as claimed, is a bioink additive for 3D bioprinting, the bioink additive comprising:
(a) nanocellulose in the form nanocellulose crystals, nanocellulose fibrils, or a combination thereof, wherein the nanocellulose crystals (if present) are characterized by a nanocrystal thermal-decomposition onset temperature of at least 290°C, and wherein the nanocellulose fibrils (if present) are characterized by a nanofibril thermal-decomposition onset temperature of at least 240°C;
(b) alginate in the form of alginic acid and/or an alginate salt; and
(c) optionally water,
wherein the alginate is ionically crosslinkable in the presence of an ionic crosslinking agent.

The principles of the present invention may be applied to crosslinkable polymers other than alginate. For example, instead of alginate or in addition to alginate, a crosslinkable natural polymer selected from gelatin, gellan gum, hyaluronic acid, fibrinogen, collagen, chitosan, or hydroxyapatite may be included in the bioink composition. Also, instead of alginate or in addition to alginate, a crosslinkable synthetic polymer selected from polycaprolactone, polylactic acid, polylactic-co-glycolic acid, polyethylene glycol, polyethylene glycol diacrylate, or polyurethane may be included in the bioink composition. Combinations of one or more crosslinkable natural polymers and/or one or more crosslinkable synthetic polymers may be included in the bioink composition. Note that crosslinking may be ionic crosslinking, covalent crosslinking, enzyme-mediated crosslinking, or a combination thereof. In the case of covalent crosslinking, the crosslinking may typically be accomplished with UV or visible light (known as photocrosslinking). Crosslinkable synthetic polymer are typically crosslinked via photocrosslinking, but crosslinkable natural polymers may be modified so that they are photocrosslinkable as well. For example, a modified natural polymer selected from methacrylated alginate, gelatin methacryloyl, methacrylated gellan gum, methacrylated hyaluronic acid, methacrylated chondroitin sulfate, methacrylamine chitosan, or methacrylated dextran may be included in the bioink composition.

Other variations of the invention that are disclosed but not presently claimed provide a method of making a bioink composition for 3D bioprinting, the method comprising:
(a) obtaining nanocellulose in the form of nanocellulose crystals, nanocellulose fibrils, or a combination thereof, wherein the nanocellulose crystals (if present) are characterized by a nanocrystal thermal-decomposition onset temperature of at least 290°C, and wherein the nanocellulose fibrils (if present) are characterized by a nanofibril thermal-decomposition onset temperature of at least 240°C;
(b) combining the nanocellulose with alginate in an aqueous solution, wherein the alginate is in the form of alginic acid and/or an alginate salt, and wherein the alginate is ionically crosslinkable in the presence of an ionic crosslinking agent;
(c) sterilizing the aqueous solution;
(d) optionally adding a plurality of viable human cells to the aqueous solution;
   and
(e) recovering the aqueous solution as a bioink composition for 3D bioprinting.

In some embodiments, the nanocellulose is present in the bioink composition at a nanocellulose concentration from 1% (w/v) to 10% (w/v). In these or other embodiments, the alginate is present in the bioink composition at an alginate concentration from 0.1% (w/v) to 5% (w/v).

Preferably, in step (a), the nanocellulose is derived from fractionation of lignocellulosic biomass in the presence of sulfur dioxide and ethanol (and water) to generate cellulose, hemicellulose, and lignin, followed by mechanical refining of the cellulose to generate the nanocellulose.

In some preferred embodiments, the nanocellulose is in the form of a combination (blend) of nanocellulose crystals and nanocellulose fibrils.

In some embodiments, in step (c), sterilizing utilizes a technique selected from the group consisting of steam sterilization, UV sterilization, ethanol (or another alcohol) sterilization, and combinations thereof. Steam sterilization may be carried out in an autoclave at a temperature of 121°C and a pressure of 100 kPa for 30 minutes, for example. UV sterilization may utilize exposure for 60 minutes to UV-C germicidal light at a wavelength of 254 nm, emitted from a germicidal lamp which produces ultraviolet (UV-C) light. This short-wave ultraviolet light disrupts DNA base pairing, causing formation of pyrimidine dimers and leads to the inactivation of bacteria and viruses. Ethanol sterilization may be carried out by immersion in 70 vol% ethanol for 30 minutes.

In step (c), the nanocellulose (or a liquid solution containing the nanocellulose) and the alginate (or a liquid solution containing the alginate) preferably are separately sterilized before being combined. Note that it is not preferred for alginate to be sterilized by steam sterilization, since alginate is thermally sensitive. Alginate is preferably UV-sterilized and/or ethanol-sterilized (or generally, alcohol-sterilized), rather than steam-sterilized. In some embodiments, nanocellulose is steam-sterilized while alginate is UV-sterilized. Alternatively, or additionally, in step (c), the entire aqueous solution may be sterilized, such as by UV-sterilizing a thin layer of the entire aqueous solution (with both nanocellulose and alginate).

For purposes of this disclosure, separately sterilizing the nanocellulose and sterilizing the alginate, followed by combining into a single aqueous solution, provides a sterilized aqueous solution since contamination would not be expected between the individual sterilization steps and the time of making the aqueous solution. The sterilizing step should, however, be conducted prior to step (d), since sterilizing would kill viable cells.

The produced bioink composition may be a cell-free bioink or a cell-laden bioink. In some embodiments, the bioink composition is a cell-laden bioink containing viable human cells, such as (but not limited to) human nasoseptal chondrocytes. Viable human cells may be present in the bioink composition at a cell concentration from 10⁵ to 10⁷ cells per milliliter of the bioink composition, for example.

Note that steps (a), (b), (c), (d), and (e) may all be carried out at the same or different locations, and at the same or different times. For example, steps (a) and (b) may form a non-sterilized solution that is stored for a period of time. Step (c) may be performed later, or potentially by a customer that purchases the nanocellulose/alginate solution. Step (d) may be performed at the same time and location as step (c), or may be done later and/or by another party. In some embodiments, all of steps (a)-(e) are carried out by one party, who sells the cell-laden bioink composition for 3D bioprinting to another party who then performs the 3D bioprinting.

Other variations that are disclosed but not presently claimed provide a method of making a 3D-printed biostructure, the method comprising:
(a) obtaining nanocellulose in the form of nanocellulose crystals, nanocellulose fibrils, or a combination thereof, wherein the nanocellulose crystals (if present) are characterized by a nanocrystal thermal-decomposition onset temperature of at least 290°C, and wherein the nanocellulose fibrils (if present) are characterized by a nanofibril thermal-decomposition onset temperature of at least 240°C;
(b) combining the nanocellulose with alginate in an aqueous solution, wherein the alginate is in the form of alginic acid and/or an alginate salt, and wherein the alginate is ionically crosslinkable in the presence of an ionic crosslinking agent;
(c) sterilizing the aqueous solution;
(d) adding a plurality of viable human cells to the aqueous solution, to form a cell-laden bioink;
(e) depositing multiple layers of the cell-laden bioink onto a substrate or surface;
(f) exposing the multiple layers of the cell-laden bioink to an ionic crosslinking agent, during step (e) and/or following step (e), to ionically crosslink the alginate; and
(g) recovering a 3D-printed biostructure containing the multiple layers of the cell-laden bioink.

In some embodiments, the nanocellulose is present in the cell-laden bioink at a nanocellulose concentration from 1% (w/v) to 10% (w/v). In these or other embodiments, the alginate is present in the cell-laden bioink at an alginate concentration from 0.1% (w/v) to 5% (w/v).

Preferably, the nanocellulose is derived from fractionation of lignocellulosic biomass in the presence of sulfur dioxide and ethanol (and water) to generate cellulose, hemicellulose, and lignin, followed by mechanical refining of the cellulose to generate the nanocellulose.

In some preferred embodiments, the nanocellulose is in the form of a combination (blend) of nanocellulose crystals and nanocellulose fibrils.

Step (c) may utilize a sterilizing technique selected from the group consisting of steam sterilization, UV sterilization, ethanol sterilization, and combinations thereof.

In some embodiments, the viable human cells are human nasoseptal chondrocytes, and the 3D-printed biostructure contains human cartilage.

In some embodiments, depositing in step (e) utilizes extrusion (i.e., extrusion-based 3D bioprinting).

The substrate or surface may be any suitable region of material onto which a first layer of bioink can be bioprinted. The substrate or surface may be biopaper, cellulose-derived paper, an inert polymer, glass, a layer of nanocellulose, or a layer of material formed from the bioink itself. In certain embodiments, there is no initial substrate; a first bioink layer is deposited as a free-standing layer in solution, or in space, with means for mechanically holding the first layer in place. The first bioink layer then acts as the substrate for additional layers.

In some embodiments, in step (f), crosslinking is performed between formation of each of the multiple layers of the cell-laden bioink. In certain embodiments, crosslinking is performed between formation of some, but not all, of the layers of the cell-laden bioink. In these or other embodiments, in step (f), crosslinking is performed after formation of all of the multiple layers of the cell-laden bioink. Note that this final step of crosslinking may replace the layer-by-layer crosslinking, or may augment such crosslinking.

The ionic crosslinking agent may include a divalent cation, such as Ca²⁺, Sr²⁺, Ba²⁺, or a combination thereof.

In some embodiments, step (f) utilizes an aerosol of the ionic crosslinking agent, wherein the aerosol is continuously or periodically exposed to the multiple layers of the cell-laden bioink. In related embodiments, step (f) utilizes a heated vapor of the ionic crosslinking agent, wherein the heated vapor is continuously or periodically exposed to the multiple layers of the cell-laden bioink. Depending on the volatility of the ionic crosslinking agent, it can be preferable to use an aerosol rather than a heated vapor, to ensure good contact between the ionic crosslinking agent and the bioink layer.

Step (f) may employ a crosslinking time from about 1 second to about 10 minutes, such as about 2, 5, 10, 15, 20, 30, or 45 seconds or about 1, 2, 3, 4, 5, 6, 7, 8, 9, or 10 minutes. In some embodiments, there is partial crosslinking with each layer, and then there is complete crosslinking after all layers are deposited. In those embodiments, crosslinking time for each layer may be about 1-30 seconds while the final crosslinking time (after all layers have been deposited) may be about 1-5 minutes, for example.

Alternatively, or additionally, step (f) may utilize immersion of one or more bioink layers into a liquid solution containing the ionic crosslinking agent. The immersion time for crosslinking may be from about 1 minute to about 1 hour, for example.

The 3D-printed biostructure, disclosed but not presently claimed, may be characterized by a porous 3D network of interconnecting compact nanocellulose particles, intimately dispersed within the alginate that is ionically crosslinked.

Preferably, the 3D-printed biostructure contains a range of nanocellulose pore sizes, such as from about 100 nanometers to about 10 microns. In some embodiments, the nanocellulose structure contains pore sizes as small as 55 nanometers and as large as 12 microns. In various embodiments, the nanocellulose structure contains at least two pore sizes selected from about 50 nm, 75 nm, 100 nm, 200 nm, 300 nm, 400 nm, 500 nm, 600 nm, 700 nm, 800 nm, 900 nm, 1 µm, 1.5 µm, 2 µm, 3 µm, 4 µm, 5 µm, 6 µm, 7 µm, 8 µm, 9 µm, 10 µm, 11 µm, 12 µm, 13 µm, 14 µm, or 15. The average pore size may be about 300 nanometers to about 3 microns, for example.

The final 3D-printed biostructure may be selected from the group consisting of an ear, a nose, a cartilaginous joint, a trachea ring, a larynx, a costal cartilage bar, or a portion thereof. The 3D-printed biostructure may be an arbitrary geometric shape.

The steps of the method of making a 3D-printed biostructure may be conducted continuously, semi-continuously, in batch, or in semi-batch.

In some variations, a 3D bioprinting process utilizes six inputs.

The first input is a computer-aided design (CAD) file, which is a blueprint of a tissue or organ, providing the geometric (spatial) information for the precise location of the bioink with living cells that are to be bioprinted. This first input may be referred to as preprocessing and may utilize magnetic resonance imaging (MRI), computerized tomography (CT) scanning, or histological studies, and/or other computational technologies. Histological studies may be performed alone or integrated with bio-imaging.

The second input is a cell-laden bioink, which is a prepared solution containing at least nanocellulose and viable cells for the tissue or organ to be printed. The bioink may include stem cells (e.g., adipose-derived stem cells, mesenchymal stem cells, cartilage stem/progenitor cells, etc.) that can differentiate into cells of the required tissue or organ, or specialized cells, such as human skin fibroblasts that can specifically produce skin tissue, or chondrocyte cells that can specifically produce cartilage, for example. Various other biocompatible materials, such as antibiotics and cell growth supplements, may be added to the bioink.

A third input, which is optional, is biopaper. Biopaper is a support material for the bioink and may contain collagen, nutrients, growth factors, and other stabilizing agents. After mixing the aforementioned components, the mixture may be chilled to a specific temperature until it achieves a gel-like consistency. Biopaper provides the environment that supports cell growth and development without impeding self-assembly. Note that bioink may be deposited onto another substrate or surface (or scaffold), other than biopaper. Also note that in certain embodiments, the biopaper itself contains nanocellulose.

A fourth input is a bioprinter, which is a device used to print out bioink in three dimensions (3D). The bioprinter may utilize a 3D bioprinting technique selected from stereolithography, extrusion-based printing, laser-assisted printing, inkjet-based printing, or nanobioprinting. In preferred embodiments, the bioprinter is an extrusion-based bioprinter. The extrusion may be via a syringe-like nozzle or a miniature extruder, for example. The bioprinter follows instructions from a CAD file, and dispenses bioink, typically from pre-filled biocartridges, to print the desired tissue or organ constructs.

A fifth input, which is optional, is one or more biocartridges. A biocartridge is a container that encloses the bioink that is to be printed. Biocartridges may be formed of any suitable material, such as plastic, and any suitable geometry, such as cylindrical vials or tubes. Biocartridges may contain pre-sterilized bioink, or non-sterilized bioink (which may be sterilized later). Biocartridges may contain cell-free bioink or cell-laden bioink. In some embodiments, biocartridges contain only a cell solution that is combined with other biocartridges contain cell-free bioink, to form a cell-laden bioink that is fed to a bioprinter, for example.

A sixth input, which is optional, is a bioreactor. A bioreactor is a physical apparatus that is configured to replicate or achieve a relevant biological environment (e.g., temperature, pH, growth factors, heat and mass transport, etc.), for further development and conditioning of cells contained in a 3D-printed biostructure, to generate tissue or an organ. Chemical and biomechanical conditioning are usually both important in a bioreactor. In a bioreactor, the 3D-printed biostructure is maintained until it becomes mature enough to be transplanted to the targeted region of a human. The bioreactor may be operated until the bioink has formed cellular spheroids, cellular cylinders, or another relevant histological form of cells. In certain embodiments, the bioreactor is an *in vivo* region within a human, i.e., when it is suitable to place the 3D-printed biostructure directly in the targeted region for potentially further growth and conditioning of cells.

In some variations of the invention, which are disclosed but not presently claimed, a process of 3D bioprinting includes a step of isolating cells from human tissue, and expanding in culture for inclusion in a cell-laden bioink. The bioink may be bioprinted in three dimensions, on a microscale (e.g., 1-100 microns) or a macroscale (e.g., millimeters or centimeters), using a 3D bioprinter to form an intermediate 3D biostructure. The intermediate 3D biostructure may be cultured in a bioreactor until tissue maturation, forming a mature 3D biostructure.

The intermediate or mature 3D biostructure may be utilized as skin for cosmetic testing, as an alternative to animal testing. The intermediate or mature 3D biostructure may be utilized as bioprinted organoids (tiny, self-organized three-dimensional tissue cultures), such as kidney or liver organoids, as an alternative to animal testing. The mature 3D biostructure may be utilized for surgical implantation tissue, such as cartilage or skin for surgical reconstruction of defects following trauma, cancer resection, or congenital conditions. The mature 3D biostructure may be utilized for surgical implantation of organs, such as kidney, liver, or heart, to overcome a shortage of donor organs.

Also disclosed but not part of the invention as claimed, is a 3D-printed biostructure comprising multiple layers, wherein each layer contains (i) nanocellulose in the form of nanocellulose crystals, nanocellulose fibrils, or a combination thereof, (ii) ionically crosslinked alginate (e.g., via divalent metal cations), and (iii) a plurality of viable human cells, wherein the 3D-printed biostructure is characterized by a porous 3D network of interconnecting compact nanorods of the nanocellulose intimately dispersed within the ionically crosslinked alginate, and wherein the viable human cells are contained on and/or in the porous 3D network.

In the 3D-printed biostructure, multiple length scales and pore sizes associated with the nanocellulose (in the bioink) are present. The nanocellulose in the 3D-printed biostructure is preferably characterized by a high thermal-decomposition onset temperature, as discussed above. The human cells may be human nasoseptal chondrocytes. The 3D-printed biostructure may contains human cartilage. The 3D-printed biostructure may be selected from the group consisting of an ear, a nose, a cartilaginous joint, a trachea ring, a larynx, a costal cartilage bar, or a portion thereof. The 3D-printed biostructure may be an arbitrary geometric shape.

The transition of the 3D-printed biostructure into functional tissue can either be undertaken *in vitro* as a bioreactor-based culture by using a variety of physiological conditions and growth factor combinations, or *in vivo* through the implantation of the 3D-printed biostructure, allowing *in situ* growth that supersedes the natural tendency for degradation. In certain embodiments, the 3D-printed biostructure is already functional, so the use of a bioreactor is not necessary.

Nanocellulose is considered to be biocompatible. In many applications, the intention is that over time (e.g., days, weeks, months, or potentially years), the nanocellulose naturally degrades and at some point is no longer present, replaced by a cell matrix (e.g., cartilage matrix). It is worth noting that nanocellulose hydrolysis *in vivo* would be expected to generate only glucose, which is non-toxic to humans, since nanocellulose is essentially a long polymer of glucose.

Applications include, but are by no means limited to, surgical and medical wound dressings, breast reconstruction, facial reconstruction, ear reconstruction, and cartilage reconstruction associated with hips or knees. Cells, tissues, organoids, and organs may be 3D-bioprinted using the disclosure herein. The 3D biostructure may be utilized to investigate signaling pathways and native cell response to toxins. Organoids and tissues may be useful to test drugs and cosmetics as a replacement for animal testing. Tissues and organs for implantation may eliminate donor site complications and overcoming the shortage of organ donors.

### Nanocellulose Production and Characterization

Nanocellulose and related materials can be produced under certain conditions including process conditions and steps associated with the AVAP^{®} biorefinery process (which is discussed in further detail below). It has been found that very high cellulose crystallinity can be produced and maintained during formation of nanofibers or nanocrystals, without the need for an enzymatic or separate acid treatment step to hydrolyze amorphous cellulose. High crystallinity can translate to mechanically strong fibers or good physical reinforcing properties, which are advantageous for bioink rheological properties and biostructure mechanical properties.

A significant techno-economic barrier for production of cellulose nanofibrils (CNF) is high energy consumption and high cost. Using sulfur dioxide (SO₂) and ethanol (or other solvent), the pretreatment disclosed herein effectively removes not only hemicelluloses and lignin from biomass but also the amorphous regions of cellulose, giving a unique, highly crystalline cellulose product that requires minimal mechanical energy for conversion to CNF. The low mechanical energy requirement results from the fibrillated cellulose network formed during chemical pretreatment upon removal of the amorphous regions of cellulose.

As intended herein, "nanocellulose" is broadly defined to include a range of cellulosic materials, including but not limited to microfibrillated cellulose, nanofibrillated cellulose, microcrystalline cellulose, nanocrystalline cellulose, and particulated or fibrillated dissolving pulp. Typically, nanocellulose as provided herein will include particles having at least one length dimension (e.g., diameter) on the nanometer scale.

"Nanofibrillated cellulose" or equivalently "cellulose nanofibrils" (CNF) or "nanocellulose fibrils" means cellulose fibers or regions that contain micron-sized particles or fibers, or both micron-sized and nanometer-sized particles or fibers. "Nanocrystalline cellulose" or equivalently "cellulose nanocrystals" (CNC) or "nanocellulose crystals" means cellulose particles, regions, or crystals that contain nanometer-sized domains. "Micron-sized" includes from 1 µm to 100 µm and "nanometer-sized" includes from 0.1 nm to 1000 nm (1 µm).

The particular size of the nanocellulose can range from the nanometer scale up to the micron scale, whether in width and/or length. Cellulose nanofibrils typically have dimensions of about 10-50 nm in width and about 500-2000 nm in length and contain both amorphous and crystalline domains of cellulose. Cellulose nanocrystals typically have width of about 3-8 nm and a length of about 100-300 nm and are predominantly crystalline. While these ranges and dimensions are typical, other particles sizes (including long fibers) may be present. Particle sizes may be measured by dynamic light scattering, atomic force microscopy, transmission-electron microscopy, small-angle X-ray scattering, or other techniques.

Generally it is beneficial to process biomass in a way that effectively separates the major fractions (cellulose, hemicellulose, and lignin) from each other. The cellulose can be subjected to further processing to produce nanocellulose. Fractionation of lignocellulosics leads to release of cellulosic fibers and opens the cell wall structure by dissolution of lignin and hemicellulose between the cellulose microfibrils. The fibers become more accessible for conversion to nanofibrils or nanocrystals. Hemicellulose sugars can be fermented to a variety of products, such as ethanol, or converted to other chemicals. Lignin from biomass has value as a solid fuel and also as an energy feedstock to produce liquid fuels, synthesis gas, or hydrogen; and as an intermediate to make a variety of polymeric compounds. Additionally, minor components such as proteins or rare sugars can be extracted and purified for specialty applications.

The following exemplary embodiments are not intended to limit the scope of the invention as claimed. The order of steps may be varied, some steps may be omitted, and/or other steps may be added. Reference herein to first step, second step, etc. is for purposes of illustrating some embodiments only.

In some embodiments, the nanocellulose is derived from a biomass source selected from the group consisting of hardwoods, softwoods, agricultural residues, unbleached chemical pulp, bleached chemical pulp, thermomechanical pulp, and combinations thereof.

In some embodiments, the nanocellulose is obtained from fractionating biomass in the presence of an acid, a solvent for lignin, and water, to generate cellulose-rich solids and a liquid phase; and then mechanically refining the cellulose-rich solids to generate the nanocellulose. In certain embodiments, the acid is sulfur dioxide and the solvent is ethanol. In certain embodiments, an AVAP^{®} process is used to make nanocellulose.

In some variations, a process for producing a nanocellulose material comprises:
(a) providing a lignocellulosic biomass feedstock;
(b) fractionating the feedstock in the presence of an acid, a solvent for lignin, and water, to generate cellulose-rich solids and a liquid containing hemicellulose and lignin;
(c) mechanically treating the cellulose-rich solids to form cellulose fibrils and/or cellulose crystals, thereby generating a nanocellulose material having a crystallinity (i.e., cellulose crystallinity) of at least 60%; and
(d) recovering the nanocellulose material.

In some embodiments, the acid is selected from the group consisting of sulfur dioxide, sulfurous acid, sulfur trioxide, sulfuric acid, lignosulfonic acid, and combinations thereof. In particular embodiments, the acid is sulfur dioxide.

The biomass feedstock may be selected from hardwoods, softwoods, forest residues, eucalyptus, industrial wastes, pulp and paper wastes, consumer wastes, or combinations thereof. Some embodiments utilize agricultural residues, which include lignocellulosic biomass associated with food crops, annual grasses, energy crops, or other annually renewable feedstocks. Exemplary agricultural residues include, but are not limited to, corn stover, corn fiber, wheat straw, sugarcane bagasse, sugarcane straw, rice straw, oat straw, barley straw, miscanthus, energy cane straw/residue, or combinations thereof. The process disclosed herein benefits from feedstock flexibility; it is effective for a wide variety of cellulose-containing feedstocks.

As used herein, "lignocellulosic biomass" means any material containing cellulose and lignin. Lignocellulosic biomass may also contain hemicellulose. Mixtures of one or more types of biomass can be used. In some embodiments, the biomass feedstock comprises both a lignocellulosic component (such as one described above) in addition to a sucrose-containing component (e.g., sugarcane or energy cane) and/or a starch component (e.g., corn, wheat, rice, etc.). Various moisture levels may be associated with the starting biomass. The biomass feedstock need not be, but may be, relatively dry. In general, the biomass is in the form of a particulate or chip, but particle size is not critical in this invention.

Mechanically treating in step (c) may employ one or more known techniques such as, but by no means limited to, milling, grinding, beating, sonicating, high-pressure impingement, high shear, or any other means to form or release nanofibrils and/or nanocrystals in the cellulose. Essentially, any type of mill or device that physically separates fibers may be utilized. Such mills are well-known in the industry and include, without limitation, Valley beaters, single disk refiners, double disk refiners, conical refiners, including both wide angle and narrow angle, cylindrical refiners, homogenizers, microfluidizers, and other similar milling or grinding apparatus. See, for example, Smook, Handbook for Pulp & Paper Technologists, Tappi Press, 1992; and Hubbe et al., "Cellulose Nanocomposites: A Review," BioResources 3(3), 929-980 (2008).

The extent of mechanical treatment may be monitored during the process by any of several means. Certain optical instruments can provide continuous data relating to the fiber length distributions and % fines, either of which may be used to define endpoints for the mechanical treatment step. The time, temperature, and pressure may vary during mechanical treatment. For example, in some embodiments, sonication for a time from about 5 minutes to 2 hours, at ambient temperature and pressure, may be utilized.

In some embodiments, a portion of the cellulose-rich solids is converted to nanofibrils while the remainder of the cellulose-rich solids is not fibrillated. In various embodiments, about 10%, 20%, 30%, 40%, 50%, 60%, 70%, 80%, 90%, 95%, 99%, or substantially all of the cellulose-rich solids are fibrillated into nanofibrils.

In some embodiments, a portion of the nanofibrils is converted to nanocrystals while the remainder of the nanofibrils is not converted to nanocrystals. In various embodiments, about 10%, 20%, 30%, 40%, 50%, 60%, 70%, 80%, 90%, 95%, 99%, or substantially all of the nanofibrils are converted to nanocrystals. During drying, it is possible for a small amount of nanocrystals to come back together and form nanofibrils.

Following mechanical treatment, the nanocellulose material may be classified by particle size. A portion of material may be subjected to a separate process, such as enzymatic hydrolysis to produce glucose. Such material may have good crystallinity, for example, but may not have desirable particle size or degree of polymerization.

Step (c) may further comprise treatment of the cellulose-rich solids with one or more enzymes or with one or more acids. When acids are employed, they may be selected from the group consisting of sulfur dioxide, sulfurous acid, lignosulfonic acid, acetic acid, formic acid, and combinations thereof. Acids associated with hemicellulose, such as acetic acid or uronic acids, may be employed, alone or in conjunction with other acids. Also, step (c) may include treatment of the cellulose-rich solids with heat. In some embodiments, step (c) does not employ any enzymes or acids.

In step (c), when an acid is employed, the acid may be a strong acid such as sulfuric acid, nitric acid, or phosphoric acid, for example. Weaker acids may be employed, under more severe temperature and/or time. Enzymes that hydrolyze cellulose (i.e., cellulases) and possibly hemicellulose (i.e., with hemicellulase activity) may be employed in step (c), either instead of acids, or potentially in a sequential configuration before or after acidic hydrolysis.

In some embodiments, the process comprises enzymatically treating the cellulose-rich solids to hydrolyze amorphous cellulose. In other embodiments, or sequentially prior to or after enzymatic treatment, the process may comprise acid-treating the cellulose-rich solids to hydrolyze amorphous cellulose.

In some embodiments, the process further comprises enzymatically treating nanocrystalline cellulose. In other embodiments, or sequentially prior to or after enzymatic treatment, the process further comprises acid-treating the nanocrystalline cellulose.

If desired, an enzymatic treatment may be employed prior to, or possibly simultaneously with, the mechanical treatment. However, in preferred embodiments, no enzyme treatment is performed to hydrolyze amorphous cellulose or weaken the structure of the fiber walls before isolation of nanofibers.

Following mechanical treatment, the nanocellulose may be recovered. Separation of cellulose nanofibrils and/or nanocrystals may be accomplished using apparatus capable of disintegrating the ultrastructure of the cell wall while preserving the integrity of the nanofibrils. For example, a homogenizer may be employed. In some embodiments, cellulose aggregate fibrils are recovered, having component fibrils in range of 1-100 nm width, wherein the fibrils have not been completely separated from each other.

The process may further comprise bleaching the cellulose-rich solids prior to step (c) and/or as part of step (c). Alternatively, or additionally, the process may further comprise bleaching the nanocellulose material during step (c) and/or following step (c). Any known bleaching technology or sequence may be employed, including enzymatic bleaching.

In some embodiments, screened pulp is bleached using the standard pulp and paper D0EpD1 bleaching sequence (chlorine dioxide, sodium hydroxide and hydrogen peroxide, and chlorine dioxide). Each step may be performed in batch or continuous mode using suitable process equipment (retention tanks, chemical mixers, etc). A water wash may be performed between the bleaching stages by diluting the pulp, such as to about 4 wt% solids with mill water and concentrating back to about 15 wt% solids in a centrifuge. Multiple washes are preferably performed after the final bleaching stage.

The nanocellulose material may include, or consist essentially of, nanofibrillated cellulose, but is not falling within the scope of the claimed subject-matter. The nanocellulose material may include, or consist essentially of, nanocrystalline cellulose, but is not falling within the scope of the claimed subject-matter. According to the presently claimed invention, the nanocellulose material includes, or consists essentially of, nanofibrillated cellulose and nanocrystalline cellulose.

In some embodiments, the crystallinity of the cellulose-rich solids (i.e., the nanocellulose precursor material) is at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86% or higher. In these or other embodiments, the crystallinity of the nanocellulose material is at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86% or higher. The crystallinity may be measured using any known techniques. For example, X-ray diffraction and solid-state ¹³C nuclear magnetic resonance may be utilized.

In some embodiments, the nanocellulose material is characterized by an average degree of polymerization from about 100 to about 1500, such as about 125, 150, 175, 200, 225, 250, 300, 400, 500, 600, 700, 800, 900, 1000, 1100, 1200, 1300, or 1400. For example, the nanocellulose material may be characterized by an average degree of polymerization from about 300 to about 700, or from about 150 to about 250. The nanocellulose material, when in the form of nanocrystals, may have a degree of polymerization less than 100, such as about 75, 50, 25, or 10. Portions of the material may have a degree of polymerization that is higher than 1500, such as about 2000, 3000, 4000, or 5000.

In some embodiments, the nanocellulose material is characterized by a degree of polymerization distribution having a single peak. In other embodiments, the nanocellulose material is characterized by a degree of polymerization distribution having two peaks, such as one centered in the range of 150-250 and another peak centered in the range of 300-700.

In some embodiments, the nanocellulose material is characterized by an average length-to-width aspect ratio of particles from about 10 to about 1000, such as about 15, 20, 25, 35, 50, 55, 60, 75, 100, 150, 200, 250, 300, 400, or 500. Nanofibrils are generally associated with higher aspect ratios than nanocrystals. Nanocrystals, for example, may have a length range of about 100 nm to 500 nm and a diameter of about 4 nm, translating to an aspect ratio of 25 to 125. Nanofibrils may have a length of about 2000 nm and diameter range of 5 to 50 nm, translating to an aspect ratio of 40 to 400. In certain exemplary embodiments, the aspect ratio for nanocrystals is about 50, and the aspect ratio for nanofibrils is about 55.

Optionally, the process further comprises hydrolyzing amorphous cellulose into glucose in step (b) and/or step (c), recovering the glucose, and fermenting the glucose to a fermentation product. Optionally, the process further comprises recovering, fermenting, or further treating hemicellulosic sugars derived from the hemicellulose. Optionally, the process further comprises recovering, combusting, or further treating the lignin.

Glucose that is generated from hydrolysis of amorphous cellulose may be integrated into an overall process to produce ethanol, or another fermentation co-product. Thus in some embodiments, the process further comprises hydrolyzing amorphous cellulose into glucose in step (b) and/or step (c), and recovering the glucose. The glucose may be purified and sold. Or the glucose may be fermented to a fermentation product, such as but not limited to ethanol. The glucose or a fermentation product may be recycled to the front end, such as to hemicellulose sugar processing, if desired.

When hemicellulosic sugars are recovered and fermented, they may be fermented to produce a monomer or precursor thereof. The monomer may be polymerized to produce a polymer, which may then be combined with the nanocellulose material to form a polymer-nanocellulose composite (e.g., a polylactide-nanocellulose composite).

In some embodiments, the nanocellulose material is at least partially hydrophobic via deposition of at least some of the lignin onto a surface of the cellulose-rich solids during step (b). In these or other embodiments, the nanocellulose material is at least partially hydrophobic via deposition of at least some of the lignin onto a surface of the nanocellulose material during step (c) or step (d). In preferred embodiments for bioink, the nanocellulose is hydrophilic and does not contain significant concentrations of lignin.

Disclosed but not part of the invention as claimed is a process for producing a nanocellulose material, the process comprising:
(a) providing a lignocellulosic biomass feedstock;
(b) fractionating the feedstock in the presence of sulfur dioxide, a solvent for lignin, and water, to generate cellulose-rich solids and a liquid containing hemicellulose oligomers and lignin, wherein the crystallinity of the cellulose-rich solids is at least 70%, wherein SO₂ concentration is from about 10 wt% to about 50 wt%, fractionation temperature is from about 130°C to about 200°C, and fractionation time is from about 30 minutes to about 4 hours;
(c) mechanically treating the cellulose-rich solids to form cellulose fibrils and/or cellulose crystals, thereby generating a nanocellulose material having a crystallinity of at least 70%; and
(d) recovering the nanocellulose material.

In some embodiments, the SO₂ concentration is from about 12 wt% to about 30 wt%. In some embodiments, the fractionation temperature is from about 140°C to about 170°C. In some embodiments, the fractionation time is from about 1 hour to about 2 hours.

In various embodiments, the nanocellulose composition is not derived from tunicates or from bacterial cellulose. In certain embodiments, the nanocellulose composition is free of enzymes or enzyme-derived fragments.

Also disclosed but not part of the invention as claimed is a process for producing a nanocellulose-containing product, the process comprising:
(a) providing a lignocellulosic biomass feedstock;
(b) fractionating the feedstock in the presence of an acid, a solvent for lignin, and water, to generate cellulose-rich solids and a liquid containing hemicellulose and lignin;
(c) mechanically treating the cellulose-rich solids to form cellulose fibrils and/or cellulose crystals, thereby generating a nanocellulose material; and
(d) incorporating at least a portion of the nanocellulose material into a nanocellulose-containing product (e.g., a nanocellulose-containing bioink composition).

The nanocellulose-containing product includes the nanocellulose material, or a treated form thereof. In some embodiments, the nanocellulose-containing product consists essentially of the nanocellulose material.

In some embodiments, step (d) comprises forming a structural object (e.g., a biostructure) that includes the nanocellulose material, or a derivative thereof.

Because there are no post-pretreatment modifications to the nanocellulose, there is potential for functionalization due to surface hydroxyl groups and negative surface charge. Without wishing to be bound by theory, the nanocellulose may be suitable for protein immobilization based on chemical conjugation and electrostatic adsorption, in order to enhance cell attachment, migration, proliferation, and differentiation.

### AVAP^{®} Biorefinery Process

The AVAP^{®} biorefinery process, which does not fall within the scope of the claimed subj ect-matter, will now be further described, in some variations.

In some embodiments, a first process step is "cooking" (equivalently, "digesting") which fractionates the three lignocellulosic material components (cellulose, hemicellulose, and lignin) to allow easy downstream removal. Specifically, hemicelluloses are dissolved and over 50% are completely hydrolyzed; cellulose is separated but remains resistant to hydrolysis; and part of the lignin is sulfonated into water-soluble lignosulfonates.

The lignocellulosic material is processed in a solution (cooking liquor) of aliphatic alcohol, water, and sulfur dioxide. The cooking liquor preferably contains at least 10 wt%, such as at least 20 wt%, 30 wt%, 40 wt%, or 50 wt% of a solvent for lignin. For example, the cooking liquor may contain about 30-70 wt% solvent, such as about 50 wt% solvent. The solvent for lignin may be an aliphatic alcohol, such as methanol, ethanol, 1-propanol, 2-propanol, 1-butanol, 2-butanol, isobutanol, 1-pentanol, 1-hexanol, or cyclohexanol. The solvent for lignin may be an aromatic alcohol, such as phenol or cresol. Other lignin solvents are possible, such as (but not limited to) glycerol, methyl ethyl ketone, or diethyl ether. Combinations of more than one solvent may be employed.

Preferably, enough solvent is included in the extractant mixture to dissolve the lignin present in the starting material. The solvent for lignin may be completely miscible, partially miscible, or immiscible with water, so that there may be more than one liquid phase. Potential process advantages arise when the solvent is miscible with water, and also when the solvent is immiscible with water. When the solvent is water-miscible, a single liquid phase forms, so mass transfer of lignin and hemicellulose extraction is enhanced, and the downstream process must only deal with one liquid stream. When the solvent is immiscible in water, the extractant mixture readily separates to form liquid phases, so a distinct separation step can be avoided or simplified. This can be advantageous if one liquid phase contains most of the lignin and the other contains most of the hemicellulose sugars, as this facilitates recovering the lignin from the hemicellulose sugars.

The cooking liquor preferably contains sulfur dioxide and/or sulfurous acid (H₂SO₃). The cooking liquor preferably contains SO₂, in dissolved or reacted form, in a concentration of at least 3 wt%, preferably at least 6 wt%, more preferably at least 8 wt%, such as about 9 wt%, 10 wt%, 11 wt%, 12 wt%, 13 wt%, 14 wt%, 15 wt%, 20 wt%, 25 wt%, 30 wt% or higher. The cooking liquor may also contain one or more species, separately from SO₂, to adjust the pH. The pH of the cooking liquor is typically about 4 or less.

Sulfur dioxide is a preferred acid catalyst, because it can be recovered easily from solution after hydrolysis. The majority of the SO₂ from the hydrolysate may be stripped and recycled back to the reactor. Recovery and recycling translates to less lime required compared to neutralization of comparable sulfuric acid, less solids to dispose of, and less separation equipment. The increased efficiency owing to the inherent properties of sulfur dioxide mean that less total acid or other catalysts may be required. This has cost advantages, since sulfuric acid can be expensive.

Additionally, and quite significantly, less acid usage also will translate into lower costs for a base (e.g., lime) to increase the pH following hydrolysis, for downstream operations. Furthermore, less acid and less base will also mean substantially less generation of waste salts (e.g., gypsum) that may otherwise require disposal.

In some embodiments, an additive may be included in amounts of about 0.1 wt% to 10 wt% or more to increase cellulose viscosity. Exemplary additives include ammonia, ammonia hydroxide, urea, anthraquinone, magnesium oxide, magnesium hydroxide, sodium hydroxide, and their derivatives.

The cooking is performed in one or more stages using batch or continuous digestors. Solid and liquid may flow cocurrently or countercurrently, or in any other flow pattern that achieves the desired fractionation. The cooking reactor may be internally agitated, if desired.

Depending on the lignocellulosic material to be processed, the cooking conditions are varied, with temperatures from about 65°C to 190°C, for example 75°C, 85°C, 95°C, 105°C, 115°C, 125°C, 130°C, 135°C, 140°C, 145°C, 150°C, 155°C, 165°C or 170°C, and corresponding pressures from about 1 atmosphere to about 15 atmospheres in the liquid or vapor phase. The cooking time of one or more stages may be selected from about 15 minutes to about 720 minutes, such as about 30, 45, 60, 90, 120, 140, 160, 180, 250, 300, 360, 450, 550, 600, or 700 minutes. Generally, there is an inverse relationship between the temperature used during the digestion step and the time needed to obtain good fractionation of the biomass into its constituent parts.

The cooking liquor to lignocellulosic material ratio may be selected from about 1 to about 10, such as about 2, 3, 4, 5, or 6. In some embodiments, biomass is digested in a pressurized vessel with low liquor volume (low ratio of cooking liquor to lignocellulosic material), so that the cooking space is filled with ethanol and sulfur dioxide vapor in equilibrium with moisture. The cooked biomass is washed in alcohol-rich solution to recover lignin and dissolved hemicelluloses, while the remaining pulp is further processed. In some embodiments, the process of fractionating lignocellulosic material comprises vapor-phase cooking of lignocellulosic material with aliphatic alcohol (or other solvent for lignin), water, and sulfur dioxide. See, for example, U.S. Patent Nos. 8,038,842 and 8,268,125.

A portion or all of the sulfur dioxide may be present as sulfurous acid in the extract liquor. In certain embodiments, sulfur dioxide is generated *in situ* by introducing sulfurous acid, sulfite ions, bisulfite ions, combinations thereof, or a salt of any of the foregoing. Excess sulfur dioxide, following hydrolysis, may be recovered and reused.

In some embodiments, sulfur dioxide is saturated in water (or aqueous solution, optionally with an alcohol) at a first temperature, and the hydrolysis is then carried out at a second, generally higher, temperature. In some embodiments, sulfur dioxide is sub-saturated. In some embodiments, sulfur dioxide is super-saturated. In some embodiments, sulfur dioxide concentration is selected to achieve a certain degree of lignin sulfonation, such as 1%, 2%, 3%, 4%, 5%, 6%, 7%, 8%, 9%, or 10% sulfur content. SO₂ reacts chemically with lignin to form stable lignosulfonic acids which may be present both in the solid and liquid phases.

The concentration of sulfur dioxide, additives, and aliphatic alcohol (or other solvent) in the solution and the time of cook may be varied to control the yield of cellulose and hemicellulose in the pulp. The concentration of sulfur dioxide and the time of cook may be varied to control the yield of lignin versus lignosulfonates in the hydrolysate. In some embodiments, the concentration of sulfur dioxide, temperature, and the time of cook may be varied to control the yield of fermentable sugars.

Once the desired amount of fractionation of both hemicellulose and lignin from the solid phase is achieved, the liquid and solid phases are separated. Conditions for the separation may be selected to minimize or enhance the reprecipitation of the extracted lignin on the solid phase. Minimizing lignin reprecipitation is favored by conducting separation or washing at a temperature of at least the glass-transition temperature of lignin (about 120°C); conversely, enhancing lignin reprecipitation is favored by conducting separation or washing at a temperature less than the glass-transition temperature of lignin.

The physical separation can be accomplished either by transferring the entire mixture to a device that can carry out the separation and washing, or by removing only one of the phases from the reactor while keeping the other phase in place. The solid phase can be physically retained by appropriately sized screens through which liquid can pass. The solid is retained on the screens and can be kept there for successive solid-wash cycles. Alternately, the liquid may be retained and solid phase forced out of the reaction zone, with centrifugal or other forces that can effectively transfer the solids out of the slurry. In a continuous system, countercurrent flow of solids and liquid can accomplish the physical separation.

The recovered solids normally will contain a quantity of lignin and sugars, some of which can be removed easily by washing. The washing-liquid composition can be the same as or different than the liquor composition used during fractionation. Multiple washes may be performed to increase effectiveness. Preferably, one or more washes are performed with a composition including a solvent for lignin, to remove additional lignin from the solids, followed by one or more washes with water to displace residual solvent and sugars from the solids. Recycle streams, such as from solvent-recovery operations, may be used to wash the solids.

After separation and washing as described, a solid phase and at least one liquid phase are obtained. The solid phase contains substantially undigested cellulose. A single liquid phase is usually obtained when the solvent and the water are miscible in the relative proportions that are present. In that case, the liquid phase contains, in dissolved form, most of the lignin originally in the starting lignocellulosic material, as well as soluble monomeric and oligomeric sugars formed in the hydrolysis of any hemicellulose that may have been present. Multiple liquid phases tend to form when the solvent and water are wholly or partially immiscible. The lignin tends to be contained in the liquid phase that contains most of the solvent. Hemicellulose hydrolysis products tend to be present in the liquid phase that contains most of the water.

In some embodiments, hydrolysate from the cooking step is subjected to pressure reduction. Pressure reduction may be done at the end of a cook in a batch digestor, or in an external flash tank after extraction from a continuous digestor, for example. The flash vapor from the pressure reduction may be collected into a cooking liquor make-up vessel. The flash vapor contains substantially all the unreacted sulfur dioxide which may be directly dissolved into new cooking liquor. The cellulose is then removed to be washed and further treated as desired.

A process washing step recovers the hydrolysate from the cellulose. The washed cellulose is pulp that may be used for various purposes (e.g., paper or nanocellulose production). The weak hydrolysate from the washer continues to the final reaction step; in a continuous digestor this weak hydrolysate may be combined with the extracted hydrolysate from the external flash tank. In some embodiments, washing and/or separation of hydrolysate and cellulose-rich solids is conducted at a temperature of at least about 100°C, 110°C, or 120°C. The washed cellulose may also be used for glucose production via cellulose hydrolysis with enzymes or acids.

In another reaction step, the hydrolysate may be further treated in one or multiple steps to hydrolyze the oligomers into monomers. This step may be conducted before, during, or after the removal of solvent and sulfur dioxide. The solution may or may not contain residual solvent (e.g. alcohol). In some embodiments, sulfur dioxide is added or allowed to pass through to this step, to assist hydrolysis. In these or other embodiments, an acid such as sulfurous acid or sulfuric acid is introduced to assist with hydrolysis. In some embodiments, the hydrolysate is autohydrolyzed by heating under pressure. In some embodiments, no additional acid is introduced, but lignosulfonic acids produced during the initial cooking are effective to catalyze hydrolysis of hemicellulose oligomers to monomers. In various embodiments, this step utilizes sulfur dioxide, sulfurous acid, sulfuric acid at a concentration of about 0.01 wt% to 30 wt%, such as about 0.05 wt%, 0.1 wt%, 0.2 wt%, 0.5 wt%, 1 wt%, 2 wt%, 5 wt%, 10 wt%, or 20 wt%. This step may be carried out at a temperature from about 100°C to 220°C, such as about 110°C, 120°C, 130°C, 140°C, 150°C, 160°C, 170°C, 180°C, 190°C, 200°C, or 210°C. Heating may be direct or indirect to reach the selected temperature.

The reaction step produces fermentable sugars which can then be concentrated by evaporation to a fermentation feedstock. Concentration by evaporation may be accomplished before, during, or after the treatment to hydrolyze oligomers. The final reaction step may optionally be followed by steam stripping of the resulting hydrolysate to remove and recover sulfur dioxide and alcohol, and for removal of potential fermentation-inhibiting side products. The evaporation process may be under vacuum or pressure, from about -0.1 atmospheres to about 10 atmospheres, such as about 0.1 atm, 0.3 atm, 0.5 atm, 1.0 atm, 1.5 atm, 2 atm, 4 atm, 6 atm, or 8 atm.

Recovering and recycling the sulfur dioxide may utilize separations such as, but not limited to, vapor-liquid disengagement (e.g. flashing), steam stripping, extraction, or combinations or multiple stages thereof. Various recycle ratios may be practiced, such as about 0.1, 0.2, 0.3, 0.4, 0.5, 0.6, 0.7, 0.8, 0.9, 0.95, or more. In some embodiments, about 90-99% of initially charged SO₂ is readily recovered by distillation from the liquid phase, with the remaining 1-10% (e.g., about 3-5%) of the SO₂ primarily bound to dissolved lignin in the form of lignosulfonates.

In a preferred embodiment, the evaporation step utilizes an integrated alcohol stripper and evaporator. Evaporated vapor streams may be segregated so as to have different concentrations of organic compounds in different streams. Evaporator condensate streams may be segregated so as to have different concentrations of organic compounds in different streams. Alcohol may be recovered from the evaporation process by condensing the exhaust vapor and returning to the cooking liquor make-up vessel in the cooking step. Clean condensate from the evaporation process may be used in the washing step.

In some embodiments, an integrated alcohol stripper and evaporator system is employed, wherein aliphatic alcohol is removed by vapor stripping, the resulting stripper product stream is concentrated by evaporating water from the stream, and evaporated vapor is compressed using vapor compression and is reused to provide thermal energy.

The hydrolysate from the evaporation and final reaction step contains mainly fermentable sugars but may also contain lignin depending on the location of lignin separation in the overall process configuration. The hydrolysate may be concentrated to a concentration of about 5 wt% to about 60 wt% solids, such as about 10 wt%, 15 wt%, 20 wt%, 25 wt%, 30 wt%, 35 wt%, 40 wt%, 45 wt%, 50 wt% or 55 wt% solids. The hydrolysate contains fermentable sugars.

Fermentable sugars are defined as hydrolysis products of cellulose, galactoglucomannan, glucomannan, arabinoglucuronoxylans, arabinogalactan, and glucuronoxylans into their respective short-chained oligomers and monomer products, i.e., glucose, mannose, galactose, xylose, and arabinose. The fermentable sugars may be recovered in purified form, as a sugar slurry or dry sugar solids, for example. Any known technique may be employed to recover a slurry of sugars or to dry the solution to produce dry sugar solids.

In some embodiments, the fermentable sugars are fermented to produce biochemicals or biofuels such as (but by no means limited to) ethanol, isopropanol, acetone, 1-butanol, isobutanol, lactic acid, succinic acid, or any other fermentation products. Some amount of the fermentation product may be a microorganism or enzymes, which may be recovered if desired.

When the fermentation will employ bacteria, such as *Clostridia* bacteria, it is preferable to further process and condition the hydrolysate to raise pH and remove residual SO₂ and other fermentation inhibitors. The residual SO₂ (i.e., following removal of most of it by stripping) may be catalytically oxidized to convert residual sulfite ions to sulfate ions by oxidation. This oxidation may be accomplished by adding an oxidation catalyst, such as FeSO4·7H₂O, that oxidizes sulfite ions to sulfate ions. Preferably, the residual SO₂ is reduced to less than about 100 ppm, 50 ppm, 25 ppm, 10 ppm, 5 ppm, or 1 ppm.

In some embodiments, the process further comprises recovering the lignin as a co-product. The sulfonated lignin may also be recovered as a co-product. In certain embodiments, the process further comprises combusting or gasifying the sulfonated lignin, recovering sulfur contained in the sulfonated lignin in a gas stream comprising reclaimed sulfur dioxide, and then recycling the reclaimed sulfur dioxide for reuse.

The process lignin separation step is for the separation of lignin from the hydrolysate and can be located before or after the final reaction step and evaporation. If located after, then lignin will precipitate from the hydrolysate since alcohol has been removed in the evaporation step. The remaining water-soluble lignosulfonates may be precipitated by converting the hydrolysate to an alkaline condition (pH higher than 7) using, for example, an alkaline earth oxide, preferably calcium oxide (lime). The combined lignin and lignosulfonate precipitate may be filtered. The lignin and lignosulfonate filter cake may be dried as a co-product or burned or gasified for energy production. The hydrolysate from filtering may be recovered and sold as a concentrated sugar solution product or further processed in a subsequent fermentation or other reaction step.

Native (non-sulfonated) lignin is hydrophobic, while lignosulfonates are hydrophilic. Hydrophilic lignosulfonates may have less propensity to clump, agglomerate, and stick to surfaces. Even lignosulfonates that do undergo some condensation and increase of molecular weight, will still have an HSO₃ group that will contribute some solubility (hydrophilic).

In some embodiments, the soluble lignin precipitates from the hydrolysate after solvent has been removed in the evaporation step. In some embodiments, reactive lignosulfonates are selectively precipitated from hydrolysate using excess lime (or other base, such as ammonia) in the presence of aliphatic alcohol. In some embodiments, hydrated lime is used to precipitate lignosulfonates. In some embodiments, part of the lignin is precipitated in reactive form and the remaining lignin is sulfonated in water-soluble form.

The process fermentation and distillation steps are intended for the production of fermentation products, such as alcohols or organic acids. After removal of cooking chemicals and lignin, and further treatment (oligomer hydrolysis), the hydrolysate contains mainly fermentable sugars in water solution from which any fermentation inhibitors have been preferably removed or neutralized. The hydrolysate is fermented to produce dilute alcohol or organic acids, from 1 wt% to 20 wt% concentration. The dilute product is distilled or otherwise purified as is known in the art.

When alcohol is produced, such as ethanol, some of it may be used for cooking liquor makeup in the process cooking step. Also, in some embodiments, a distillation column stream, such as the bottoms, with or without evaporator condensate, may be reused to wash cellulose. In some embodiments, lime may be used to dehydrate product alcohol. Side products may be removed and recovered from the hydrolysate. These side products may be isolated by processing the vent from the final reaction step and/or the condensate from the evaporation step. Side products include furfural, hydroxymethyl furfural (HMF), methanol, acetic acid, and lignin-derived compounds, for example.

The glucose may be fermented to an alcohol, an organic acid, or another fermentation product. The glucose may be used as a sweetener or isomerized to enrich its fructose content. The glucose may be used to produce baker's yeast. The glucose may be catalytically or thermally converted to various organic acids and other materials.

When hemicellulose is present in the starting biomass, all or a portion of the liquid phase contains hemicellulose sugars and soluble oligomers. It is preferred to remove most of the lignin from the liquid, as described above, to produce a fermentation broth which will contain water, possibly some of the solvent for lignin, hemicellulose sugars, and various minor components from the digestion process. This fermentation broth can be used directly, combined with one or more other fermentation streams, or further treated. Further treatment can include sugar concentration by evaporation; addition of glucose or other sugars (optionally as obtained from cellulose saccharification); addition of various nutrients such as salts, vitamins, or trace elements; pH adjustment; and removal of fermentation inhibitors such as acetic acid and phenolic compounds. The choice of conditioning steps should be specific to the target product(s) and microorganism(s) employed.

In some embodiments, hemicellulose sugars are not fermented but rather are recovered and purified, stored, sold, or converted to a specialty product. Xylose, for example, can be converted into xylitol.

A lignin product can be readily obtained from a liquid phase using one or more of several methods. One simple technique is to evaporate off all liquid, resulting in a solid lignin-rich residue. This technique would be especially advantageous if the solvent for lignin is water-immiscible. Another method is to cause the lignin to precipitate out of solution. Some of the ways to precipitate the lignin include (1) removing the solvent for lignin from the liquid phase, but not the water, such as by selectively evaporating the solvent from the liquid phase until the lignin is no longer soluble; (2) diluting the liquid phase with water until the lignin is no longer soluble; and (3) adjusting the temperature and/or pH of the liquid phase. Methods such as centrifugation can then be utilized to capture the lignin. Yet another technique for removing the lignin is continuous liquid-liquid extraction to selectively remove the lignin from the liquid phase, followed by removal of the extraction solvent to recover relatively pure lignin.

Lignin can be used as a fuel. As a solid fuel, lignin is similar in energy content to coal. Lignin can act as an oxygenated component in liquid fuels, to enhance octane while meeting standards as a renewable fuel. The lignin produced herein can also be used as polymeric material, and as a chemical precursor for producing lignin derivatives. The sulfonated lignin may be sold as a lignosulfonate product, or burned for fuel value.

### EXAMPLES

The bioprintability of crystalline, fibrillated, and blend formulations of nanocellulose was determined by assessing resolution (grid-line assay) as well as post-printing shape fidelity and rheology (elasticity, viscosity, and shear-thinning characteristics) and comparing these to pure-alginate bioinks. An optimized nanocellulose-alginate bioink was bioprinted with human nasoseptal chondrocytes to determine cytotoxicity, metabolic activity, and biostructure topography.

As will be shown in these Examples, the disclosed nanocellulose-alginate bioink compositions demonstrated a high degree of shear thinning with reversible stress-softening behavior which contributed to post-printing shape fidelity. A blend of crystal and fibril nanocellulose-containing bioink exhibited nanoscale roughness as well as microscale roughness for cellular survival and differentiation, and maintained the most stable construct volume in culture. Human nasoseptal chondrocytes demonstrated high metabolic activity post-printing and adopted a rounded chondrogenic phenotype after prolonged culture.

Kyle et al., "Characterization of pulp derived nanocellulose hydrogels using AVAP® technology", Carbohydrate Polymers 198 (2018) 270-280 and Jessop et al., "Printability of pulp derived crystal, fibril and blend nanocellulose-alginate bioinks for extrusion 3D bioprinting", 2019 Biofabrication in press, available at https://doi.org/10.1088/1758-5090/ab0631.

### Extrusion 3D Bioprinting Set-up

A custom-built extrusion bioprinter with a variable speed control syringe driver mounted on a motor-driven XYZ system featuring custom RepRap firmware was used to print 3D structures from nanocellulose/alginate hydrogel bioinks. Bioink was loaded into 5 mL syringes (Sterile BD Plastipak slip tip) and extruded through 610 µm precision nozzles (Adhesive Dispensing Ltd). Slic3r (open source software) allowed adjustment of print settings to suit the individual bioinks; including print-head movement speed, extrusion speed, layer height, infill pattern, and density. The 3D model was sliced and exported as G-code to command the bioprinter. Initial calibration of the printer and software resulted in a minimum prefabrication layer of 1.7 mm to allow for continuous, uniform printing. Sterile printing was achieved in a class II laminar flow hood following cleaning of all external bioprinter components using 70% ethanol and UV treatment for 60 minutes.

### Bioink Preparation and Optimization

Nanocellulose particles were produced as an aqueous slurry from raw wood chip biomass using patented AVAP^{®} technology which fractionates biomass into cellulose, hemicelluloses, and lignin using ethanol, sulfur dioxide, and water, with morphology controlled by the time and temperature of the fractionation. The final nanocellulose formulations are hydrophilic BioPlus^{®} Cellulose Nanocrystals gel (CNC, 3 wt% nanocellulose in water), hydrophilic BioPlus^{®} Cellulose Nanofibrils gel (CNF, 6 wt% nanocellulose in water), and hydrophilic BioPlus^{®} blend gel (NCB, 3 wt% nanocellulose in water).

CNC, NCB, and CNF were centrifuged at 1500g for 5 minutes to remove any residual chemicals and lignin fragments and then steam-sterilized in an autoclave (100 kPa, 121°C, 30 minutes). Alginic acid sodium salt (medium viscosity, from brown algae, Sigma-Aldrich, Poole, UK) was UV-sterilized for one hour prior to being dissolved in sterile culture medium to make up 0.625, 1.25, 2.5, 5, 7.5 and 10% alginate concentrations (w/v). Excess water was removed and replaced with exactly the same volume of alginate-containing hydrogel. One part alginate was combined with four parts nanocellulose to create CNC-AG, NCB-AG and CNF-AG bioinks, which are crosslinkable with calcium chloride (CaCl₂) following bioprinting. The concentrations of the experimental nanocellulose-alginate bioinks were 0.16%, 0.31%, 0.63%, 1.25%, 1.88%, 2.5% (w/v) of alginate and either 3% (w/v) for cellulose nanocrystals and for cellulose nanocrystal/nanofibril blend, 6% (w/v) for cellulose nanofibrils.

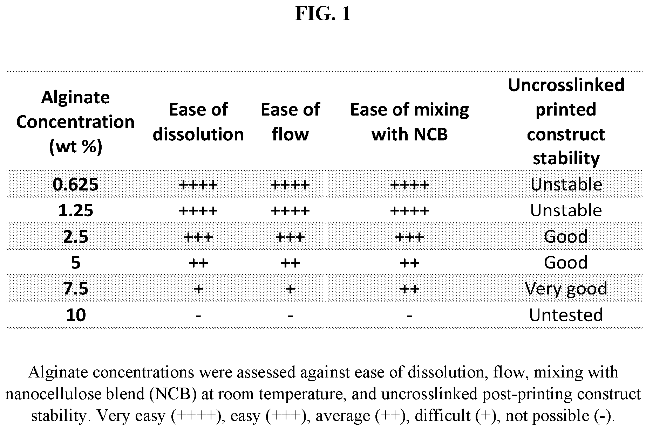

Alginate solutions from 0.625% to 10% (w/v) were qualitatively assessed against four criteria set to define optimal bioink preparations; ease of dissolution, ease of flow at room temperature, ease of mixing with nanocellulose, and structural retention when printed with nanocellulose. FIG. 1 shows the results of the qualitative analysis.

### Bioink Crosslinking Optimization

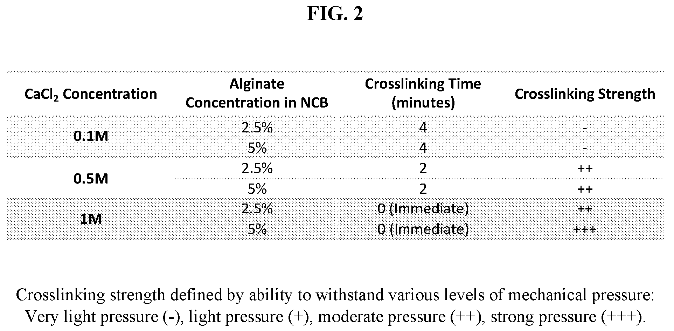

Different concentrations (0.1 M, 0.5 M, and 1 M) of aerosolized CaCl₂ solutions (anhydrous CaCl₂ powder, Sigma-Aldrich, Poole, UK, dissolved in distilled water) were tested for crosslinking time from print completion to cessation of structural changes on application of an indentation force. All solutions were sterilized and filtered by a 0.22 µm cell filter (Merck Millipore, Watford, UK) and stored in an incubator at 37°C before use. Cylindrical constructs measuring 27 mm in diameter and 7 single layers (11.9 mm) in height were printed using alginate concentrations of 2.5% and 5% (w/v) mixed with nanocellulose in a 1:4 weight ratio, as described above. Crosslinking was performed by exposing each layer of bioprinted bioink to aerosolized CaCl₂. FIG. 2 shows a quantitative analysis of crosslinking time (minutes) and a qualitative analysis of crosslinking strength.

### Printability Testing: Resolution

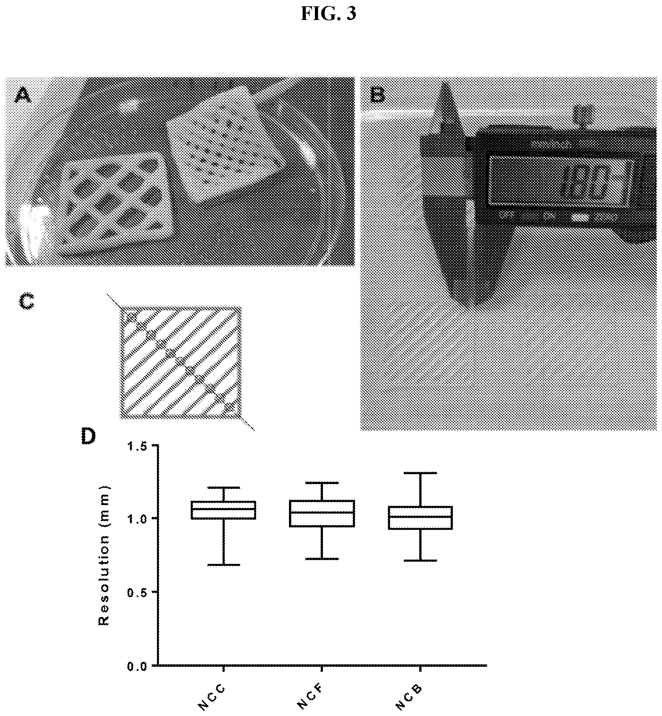

Print resolution using the three different formulations of nanocellulose (CNC-AG, CNF-AG, and NCB-AG) was tested by printing 40 × 40 mm square grids a single layer tall (1.7 mm) with 27% rectilinear infill and crosslinked with 0.5 M CaCl₂ (FIG. 3), adapted from Markstedt et al., "3D Bioprinting Human Chondrocytes with Nanocellulose-Alginate Bioink for Cartilage Tissue Engineering Applications", Biomacromolecules 2015, 16, 1489-1496. Grid line thickness was measured at the halfway points of the infill diagonal lines using a digital caliper (inset B of FIG. 3). Shape fidelity of bioprinted and crosslinked structures using the three formulations CNC-AG, CNF-AG, and NCB-AG was assessed by bioprinting 27 mm diameter cylinders with 7-layer height (11.9 mm) and measuring diameter and height immediate post-printing, at 24 hr and 72 hr following immersion in culture media at 37°C.

FIG. 3 shows the results of a grid thickness assay to test bioprinting resolution for nanocellulose-containing bioinks. Inset A of FIG. 3 shows crosslinked lattices printed using NCB-AG, revealing structural retention on agitation with a spatula. Inset B of FIG. 3 shows an electronic calliper measurement of filaments printed using NCB-AG. Inset C of FIG. 3 is a schematic diagram to illustrate measurement points of the construct for resolution testing. Inset D of FIG. 3 shows filament thickness for CNC-AG, CNF-AG, and NCB-AG formulations. Data for filament thickness is expressed as the mean, and error bars indicate standard deviation. Statistical differences were calculated by one-way analysis of variance (ANOVA); *p* = 0.424, pooled data from four constructs, each with *n =* 11. In this patent application, *p* is the probability for a given statistical model that, when the null hypothesis is true, the statistical summary (such as the sample mean difference between two compared groups) would be greater than or equal to the actual observed results, and *n* is the number of replicates.

### Printability Testing: Shape Fidelity

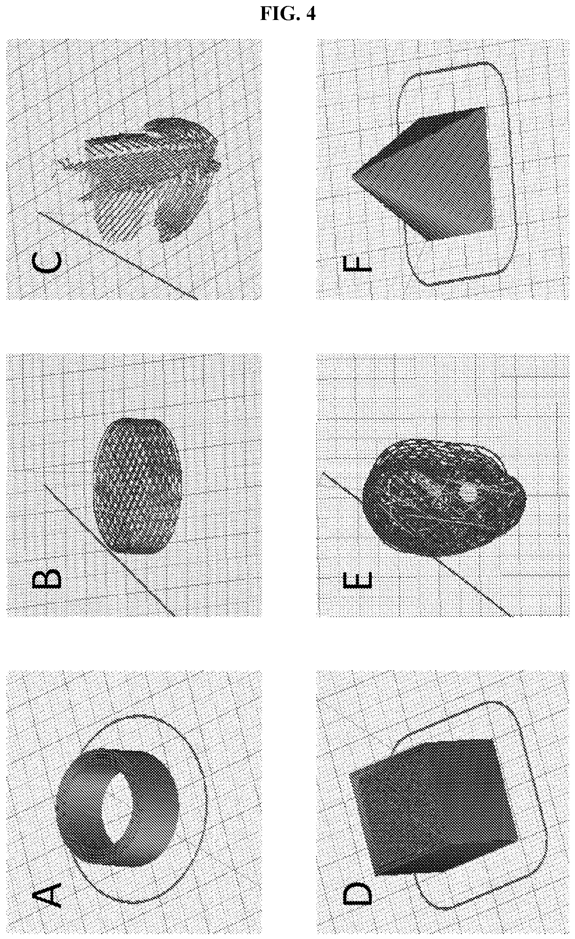

Complex structure testing utilized 3D geometrical shapes and anatomical STL models of auricular cartilage, obtained from an online open source repository, BodyParts3D^{©} (The Database Center for Life Science, licensed under CC Attribution-Share Alike 2.1 Japan (http://lifesciencedb.jp/bp3d/) (see FIG. 4). Cylindrical shapes were created using Microsoft 3D Builder software (Microsoft Corporation, New Mexico, USA) and exported in STL file format for 3D bioprinting. The volume was calculated by measuring the diameter (D) and height (h) of the 3D printed cylindrical constructs using V = πDh to assess post-printing shape fidelity after 24 and 48 hours under culture conditions. FIG. 4 shows G-code in graphical format of the shapes used for complex structure testing. In FIG. 4, inset A is a hollow cylinder, inset B is a cylinder, inset C is nasal cartilage, inset D is a cube, inset E is a right ear, and inset F is a 4-sided pyramid.

### Printability Testing: Rheology

The rheological properties of bioink mixtures (alginate 1.25%/2.5%/5% (w/v) and nanocellulose mixtures with alginate 1.25%, 2.5% and 5% (w/v) to create CNC-AG, NCB-AG, and CNF-AG bioinks with overall alginate concentrations of 0.25%, 0.5% and 1% (w/w) respectively) were measured using an AR-G2 (TA instruments, UK) Controlled Stress Rheometer fitted with a 40 mm diameter parallel-plate geometry. Solutions were mixed on a rolling rocker for about 15 min before sample loading. Prior to each experiment, the zero gap was set on the rheometers and the geometry was calibrated using rotational mapping.

Approximately 0.65 mL of sample was carefully loaded using a spatula onto the center of the lower plate of the rheometer and the upper plate was gradually lowered onto the sample until the gap was totally filled (gaps ranged from 350-500 µm). Any excess sample around the edge of the geometry was trimmed using a spatula. The normal force measured at the lower plate was set at a maximum of 0.1 N to ensure that any mechanical damage to the sample during the gap-setting procedure was minimized. The rheometer lower plate was controlled at a temperature of 22°C and a low viscosity silicon oil (Fisher Scientific: Brookfield, Viscosity standard 0.920 specific gravity silicone oil 5 mPa·s) was used to surround the outer edges of the sample, in order to reduce sample evaporation.

Following a sample equilibration period of 2 minutes, a frequency sweep (0.1 to 10 Hz) was performed at a constant stress of 0.5 Pa. Each measurement was within the linear viscoelastic range of the sample as confirmed by stress sweeps

(data not shown). The values of storage modulus (G') and loss modulus (G") were recorded over the entire frequency range employed. Following the completion of the frequency sweep, the sample was allowed to equilibrate for a period of 10 seconds before a shear flow ramp was carried out over logarithmically increasing shear rates from 0.1-100 s⁻¹ for a period of 2 minutes. Both frequency sweep and shear flow ramp experiments were repeated at least three times for each sample.

### 3D Bioprinting with Human Nasoseptal Chondrocytes: Cell Culture, Encapsulation, and Bioprinting

Chondrocytes were isolated from human nasoseptal cartilage samples obtained after informed consent (IRAS ID 99202) during routine septorhinoplasties. Cartilage tissue was minced into 1 mm³ pieces and digested by 0.4% pronase (Roche, West Sussex, UK) in culture media for 1 hour at 37°C (5% CO₂) with gentle agitation, followed by digestion with 0.2% collagenase type I for 18 hours. The solution was filtered through a 40 µm cell strainer (VWR, Leicestershire, UK) and then centrifuged at 500g for 5 minutes to replace the enzyme mixture with culture media. Cells were grown in 5% CO₂ at 37°C and culture medium was changed every 2-3 days. Culture medium consisted of Dulbecco's Modified Eagle Medium without glucose (Sigma-Aldrich, Poole, UK) supplemented with 10% fetal bovine serum (Sigma-Aldrich, Poole, UK), 100 µg/mL penicillin and 100 U/mL streptomycin (Sigma-Aldrich, Poole, UK), 1 mM glucose (Sigma-Aldrich, Poole, UK), and 0.1% non-essential amino acids (Thermo Fisher Scientific, Paisley, UK). Cells were passaged using 0.05% trypsin-EDTA (Thermo Fisher Scientific, Paisley, UK) when they reached 80-90% confluency. The number of total and viable cells was estimated by 0.4% trypan blue staining using the Invitrogen^{™} Countess^{™} II automated cell counter (Thermo Fisher Scientific, Paisley, UK).

Passages 5-7 were chosen for subsequent experiments because they yielded the adequate number of chondrocytes for extrusion 3D bioprinting using 2 × 10⁶ cells per mL of bioink. Immediately prior to bioprinting of cell-laden bioink, cell suspensions were drawn up into a syringe and gently mixed with a syringe of nanocellulose/alginate cell-free bioink using a two-way tap under sterile conditions for one minute to ensure uniform distribution within the cell-laden bioink. The cell-laden bioink was printed into layers, in an atmosphere containing aerosolized CaCl₂, with a crosslinking time of about 10 seconds between each layer. Immediately post-printing (all layers completed), the cell-bioink constructs were washed in culture medium to remove excess CaCl₂ before culturing in the incubator.

### 3D Bioprinting with Human Nasoseptal Chondrocytes: Biocompatibility

A LIVE/DEAD^{®} Cell Viability Kit (Thermo Fisher Scientific, Paisley, UK) was used for assessment of cell viability, according to the manufacturer's instructions, at 24 and 48 hours after bioprinting. The samples were stained with 2 µM calcein AM (green fluorescent dye) and 1 µM ethidium homodimer-1 (red fluorescent dye). Labeling was examined using confocal microscopy (Zeiss LSM 710 inverted confocal microscope), where green labels represent live cells and red labels indicate dead cells. Images were taken from at least six different areas of three bioprinted constructs for each condition. The number of live and dead cells were counted using NIH ImageJ software, and cell viability was expressed as the percentage of the number of live cells to total cells.

Cell metabolic activity was quantified using 10% (v/v) AlamarBlue^{®} cell viability reagent (Thermo Fisher Scientific, Paisley, UK) at 4, 12, and 24 hours after bioprinting. The fluorescence was measured at 530-560 nm excitation wavelength and 590 nm emission wavelength with a plate reader (POLARstar Omega spectrophotometer, BMG LABTECH, Ortenberg, Germany). Matched concentrations of cells in media were used as controls. The cross reactivity of AlamarBlue with nanocellulose in the medium was also tested. The optical density (OD) values were normalized against that at *t* = 0. The cell viability was determined by plotting fluorescence emission intensity versus cell concentration.

### 3D Bioprinting with Human Nasoseptal Chondrocytes: Cytotoxicity

Cytotoxity of NCB bioink was assessed using a lactose dehydrogenase (LDH) cytotoxicity assay. The plate containing cells with NCB-AG (*n* = 6) as well as cells only (*n =* 6), media only (*n =* 3) and NC controls (*n* = 3) were incubated at 37°C, 5% CO₂ overnight. Then 50 µL of each sample medium was transferred to a 96-well plate and mixed with 50 µL reaction mixture for 30 minutes at room temperature, protected from light, at which point stop solution was added. The absorbance measurements (at 490 nm and 680 nm) were used to determine LDH activity.

### 3D Bioprinting with Human Nasoseptal Chondrocytes: SEM Analysis

Bioprinted and crosslinked cellular constructs grown for 3 weeks in culture were washed three times with 50 mM sodium cacodylate-HCl buffer solution (pH 7.2-7.4, SPI Supplies) at 10 to 20 minute intervals to remove excess salt. The samples were fixed overnight in 2% glutaraldehyde (Sigma Aldrich, UK) and dehydrated with a series of graded concentrations (30% to 100%) of ethanol. The dehydrated sample was then rinsed with 50% hexamethyldisilazane solution (HMDS) in 100% ethanol for 10 minutes in a fume hood and then three times in 100% HMDS and left overnight to dry. The sample was coated with a thin layer of gold (~15 nm layer thickness) using sputter coating and was imaged using scanning electron microscopy (Hitachi 4800s).

### 3D Bioprinting with Human Nasoseptal Chondrocytes: Compression Testing

Mechanical tests were performed on bioprinted NCB-AG cylinder discs (5 mm diameter and 4 mm height), crosslinked with 0.5 M of CaCl₂ for 5 min and incubated in phosphate-buffered saline (Thermo Fisher Scientific, Paisley, UK) at room temperature for 1 hour to reach swelling equilibrium, using the BOSE ElectroForce^{®} 3200 mechanical loading machine (Bose Corporation, ElectroForce^{®} Systems Group, Minnesota, USA). The compressive strength of the samples was tested at 1 Hz. The compression (mm) and load (N) were recorded and Young's modulus was determined as the slope of the linear region of the stress/strain curve: Young's Modulus (N/m² or Pa) = Stress (Pa)/Strain.

Quantitative results are expressed as mean ± standard deviation (SD), and the statistical analyses were performed using the GraphPad Prism 6.0 (GraphPad Software, La Jolla, CA, USA). Statistical analyses for single comparisons were performed using the Mann-Whitney U-test for non-Gaussian distributions and the t-test for Gaussian distributions. Multiple comparisons were performed by one-way ANOVA, followed by a Tukey's post hoc test. The number of biological replicates is indicated in each figure legend. A value ofp < 0.05 was considered statistically significant.

### Nanocellulose Bioink Formulation and Crosslinking Optimization

0.625% (w/v) alginate solution exhibited superior properties with regards to dissolution and mixing but quickly collapsed at room temperature post-printing. 7.5% and 10% (w/v) alginate solutions were difficult to dissolve and too viscous to mix evenly with nanocellulose. Concentrations of 1.25%, 2.5% and 5% (w/v) were found to have suitable viscosities and mixing potential and went on to rheological testing. The alginate concentration of 2.5% (w/v) was chosen for further experiments due its ability to balance ease of dissolution and mixing with uncrosslinked structure stability post-printing (FIG. 1).

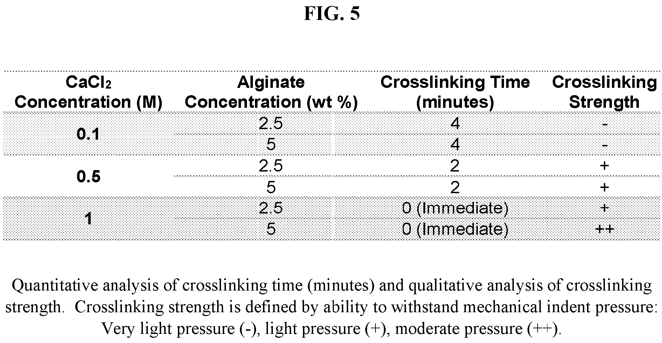

Crosslinking optimization with different CaCl₂ concentrations is summarized in FIG. 5. 1 M CaCl₂ was crosslinked in the shortest time (immediately, compared to 2 minutes for 0.5 M and 4 minutes for 0.1 M), with final constructs able to withstand larger mechanical force compared to 0.5 M and 0.1 M. However, it was found that the immediate crosslinking time of 1 M CaCl₂ resulted in crosslinking of the filament during layer deposition, causing dragging and ultimately spoiling the final printed structure. FIG. 3 (inset A) illustrates crosslinked lattice structures using the preferred concentration of 0.5 M CaCl₂ withstanding agitation using a spatula.

Transmission electron microscopy (TEM) was utilized to assess crosslinking, fiber entanglement, and porosity. Each sample (2 mg) was dispersed in 5 mL of deionized water and sonicated for 30 minutes. After sonication, 50 µL of the sample was immediately taken and further dispersed into 1 mL of deionized water to prevent coalescence. This solution (10 µL) was added to 300-mesh copper grids coated with lacey carbon film. The grid was allowed to air dry prior to staining with a 1.5% uranyl acetate solution. For staining, a drop of the uranyl acetate solution was placed on a Parafilm^{®} strip (Bemis Company, Inc., Neenah, WI, USA) and the grid inverted onto the droplet for a few seconds. The samples were then allowed to air dry. Analysis was performed on a Jeol 2100 JEM operating at 200 kV.

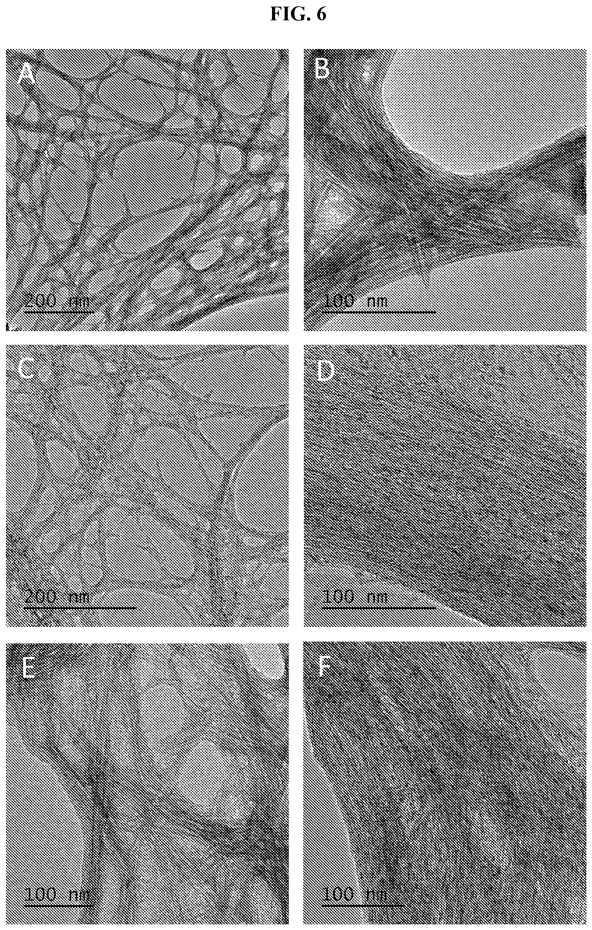

FIG. 6 shows TEM images of nanocellulose-alginate bioinks before and after crosslinking. In particular, panels A and B shows CNC-AG bioink before and after crosslinking, respectively; panels C and D show CNF-AG bioink before and after crosslinking, respectively; and panels E and F show NCB-AG before and after crosslinking, respectively. In all cases (i.e., panels B, D, and F of FIG. 6), crosslinking is achieved using aerosolized 0.5 M CaCl₂.

All three formulations displayed varying degrees of nanofiber entanglements and extensive porous networks as seen on TEM imaging prior to crosslinking due to agglomeration (FIG. 6, panels A, C, and E). NCB-AG shows fibrillar entanglements of nanofibrils interspersed with nanocrystals (FIG. 6, panel E). Without being limited by speculation, it is believed that on crosslinking with CaCl₂, the alginate pulls the nanofibrils and nanocrystals together to reduce voids within the material, thereby producing a firm structure that can be manipulated and can withstand cell culture conditions (FIG. 6, panels B, D, and F).

### 3D Bioprinting of Macrostructures Using Nanocellulose-Containing Bioinks

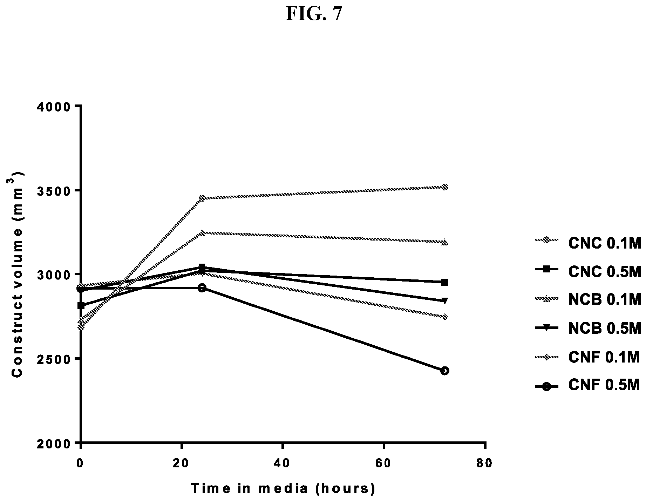

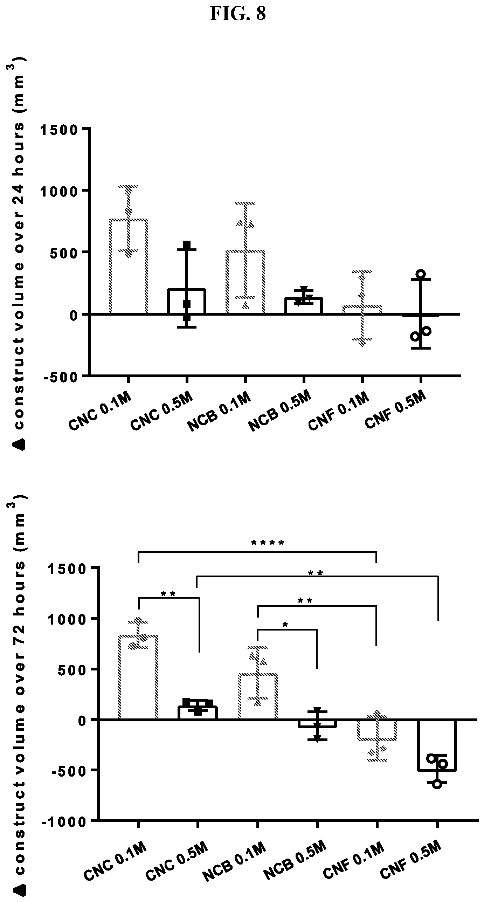

FIGS. 7 and 8 show bioprinted construct swelling properties. FIG. 7 provides a comparison of cylindrical construct volume bioprinted using three different bioink formulations (CNC-AG, NCB-AG, and CNF-AG) crosslinked with either 0.1 M or 0.5 M CalCl₂. FIG. 8 shows the change in construct volume relative to time 0 at 24 and 72 hours. Data is expressed as the mean ± SD (*n* = 3 for all groups). Statistical differences are calculated by one-way ANOVA with Tukey's multiple comparison post-hoc test. In FIG. 8, * denotes *p* < 0.05, ** denotes *p* < 0.01, and **** denotes *p* < 0.001.

NCB-AG had better resolution demonstrated by the median filament thickness of 1.01 mm compared to 1.07 mm and 1.04 mm for CNC-AG and CNF-AG, respectively (FIG. 3, inset D); however, this was not found to be statistically significant (p = 0.42, one-way ANOVA). Crosslinked CNC-AG and NCB-AG cylindrical constructs swelled, especially at low crosslinker concentrations, over 24 hours under culture conditions (in media at 37°C), but these changes were not statistically significant (FIG. 7 and FIG. 8). After 72 hours, there was a significant increase in volume for CNC-AG (697.6 mm³, *p* = 0.003) and NCB-AG (523.9 mm³, *p* = 0.021) constructs crosslinked with 0.1 M versus 0.5 M CalCl₂ concentrations, whereas constructs printed using CNF-AG decreased in volume, with 0.5 M CaCl₂ crosslinked constructs have a greater reduction in volume (488.3 mm³) than 0.1 M CaCl₂ crosslinked constructs (186.8 mm³) from their original size (FIG. 8). NCB-AG constructs crosslinked with 0.5 M CalCl₂ were the most stable under culture conditions, with a reduction of 62.3 mm³ from their original size.

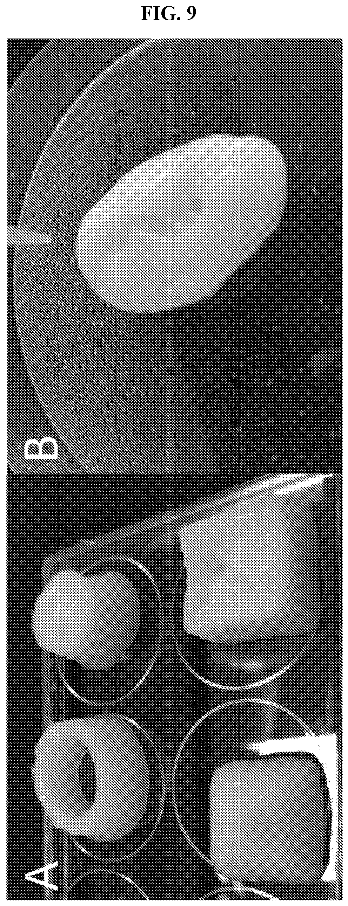

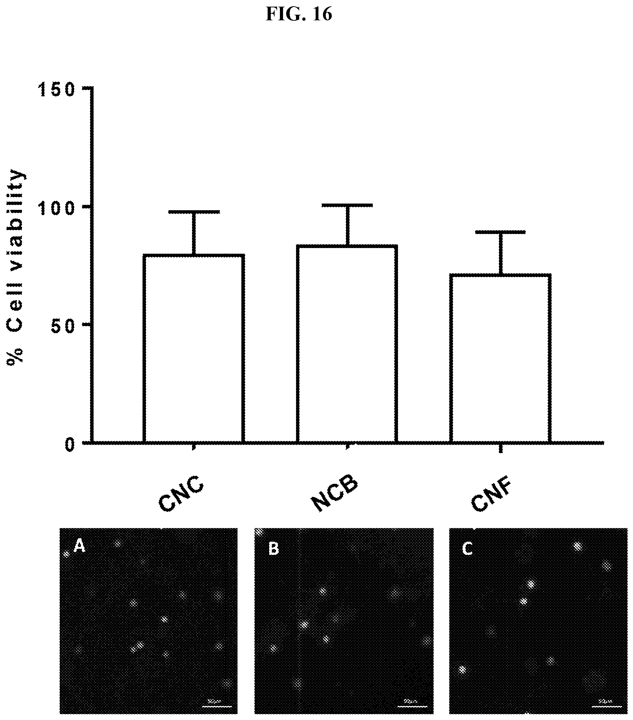

Since NCB-AG crosslinked with 0.5 M CaCl₂ demonstrated the most stable construct volume under culture conditions, this was the formulation chosen for complex shape testing, including hollow cylinders and anatomical auricular cartilage (FIG. 9) and biocompatibility experiments (FIGS. 16-19).

FIG. 9 shows geometric structures printed using NCB-AG bioink for complex shape testing. In panel A of FIG. 9, the geometric structures are a hollow cylinder (top left), cylinder (top right), pyramid (bottom left), and cube (bottom right). In panel B of FIG. 9, the geometric structure is a right ear.

### Rheological Properties of BioPlus^{®} Nanocellulose Enables Bioprinting

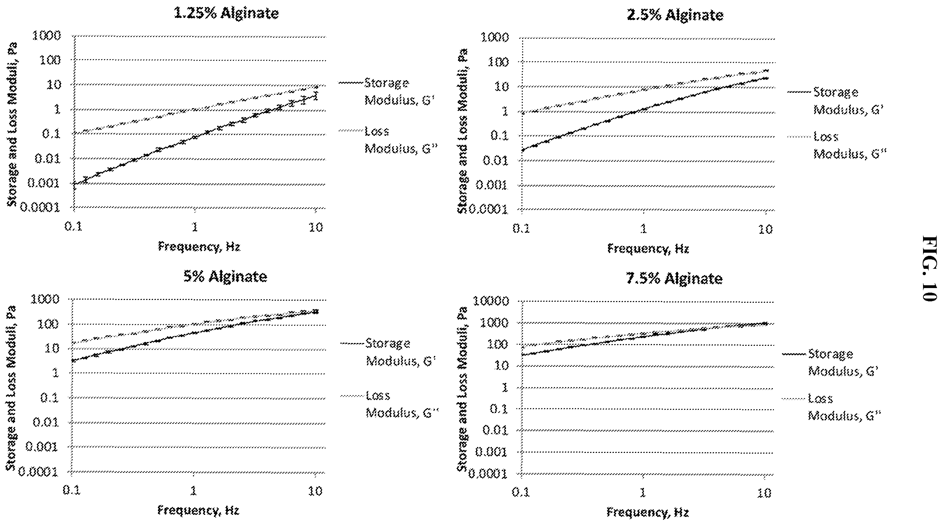

FIG. 10 shows rheological properties of bioinks containing alginate but not nanocellulose (referred to as "pure alginate" for convenience, noting the balance is primarily water). In FIG. 10, G' is storage modulus (dark grey), and G" is loss modulus (light grey) at varying concentrations of alginate. Data is expressed as the mean ± SD (*n* = 5 for all groups).

Pure alginate bioinks showed increasing G' and G" with increasing frequency (FIG. 10). The mean loss moduli (G") were consistently higher than mean storage moduli (G') for all alginate concentrations (1.25, 2.5, 5 and 7%, w/v), demonstrating a dominance of viscous over elastic properties in pure alginate bioinks.

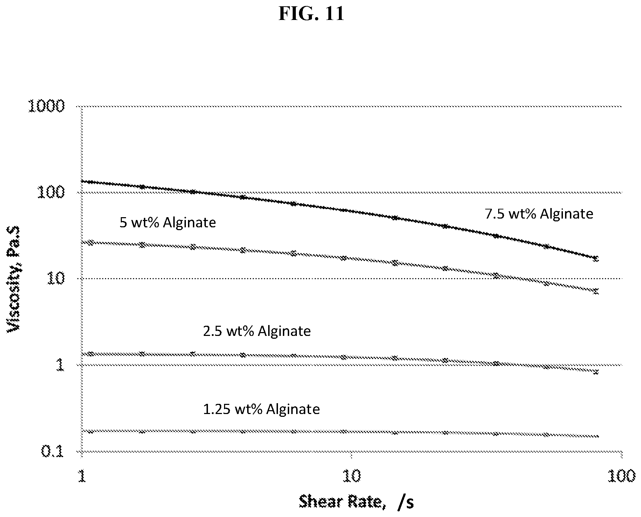

Shear rate ramps were performed to investigate the flow properties of bioinks by measuring the viscosity as a function of increasing shear rate. FIG. 11 shows viscosity of pure-alginate bioinks as a function of shear rate. Data is expressed as the mean ± SD *(n* = 5 for all groups). FIG. 11 reveals limited shear thinning behavior at 5% and 7.5% (w/v) and near Newtonian behaviour at 1.25% and 2.5% (w/v) alginate concentrations of non-nanocellulose-containing bioinks over the entire shear rates studied.

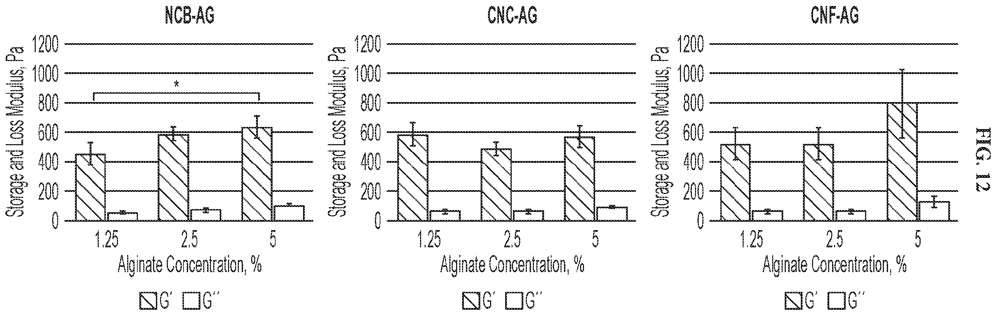

By contrast, the mean G' for all nanocellulose-containing bioinks is greater than the G" over all frequencies studied, indicating a dominance of elasticity (unlike alginate bioinks) and demonstrating strong interconnecting networks between nanostructures (FIG. 12). FIG. 12 shows rheological properties of nanocellulose-alginate bioinks at 1 Hz. In FIG. 12, G' is storage modulus (dark grey), and G" is loss modulus (light grey) for blend (NCB), crystal (CNC), and fibril (CNF) nanocellulose-containing bioinks, at varying concentrations of alginate and nanocellulose. Data is expressed as the mean ± SD *(n* = 3 for all groups). Statistical differences were calculated by one-way ANOVA with Tukey's multiple comparison post-hoc test (* denotes *p* < 0.05).

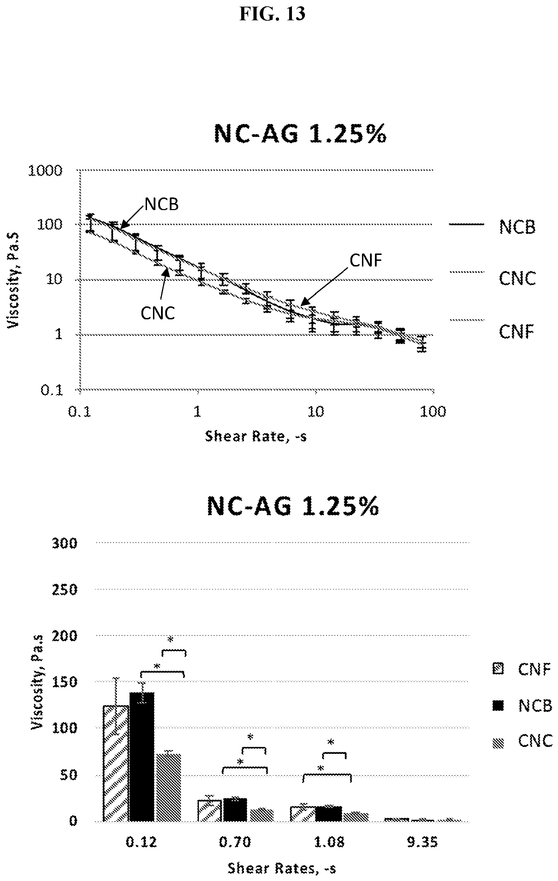

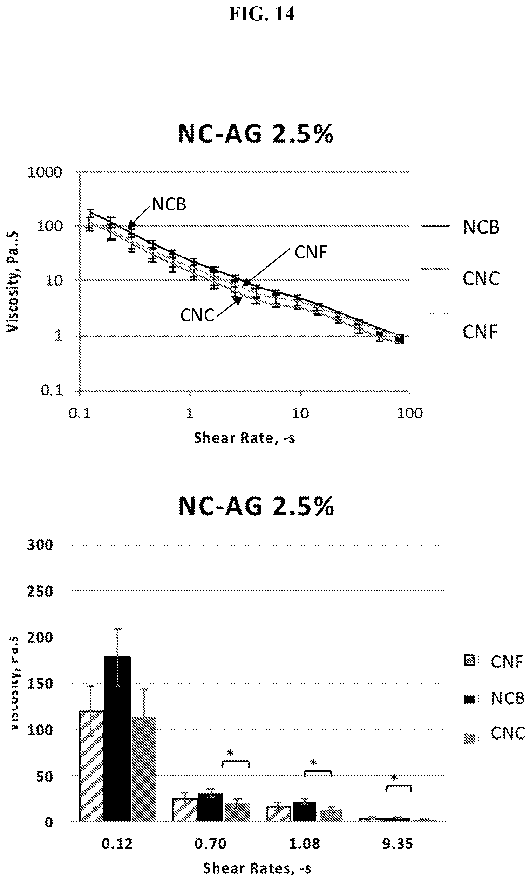

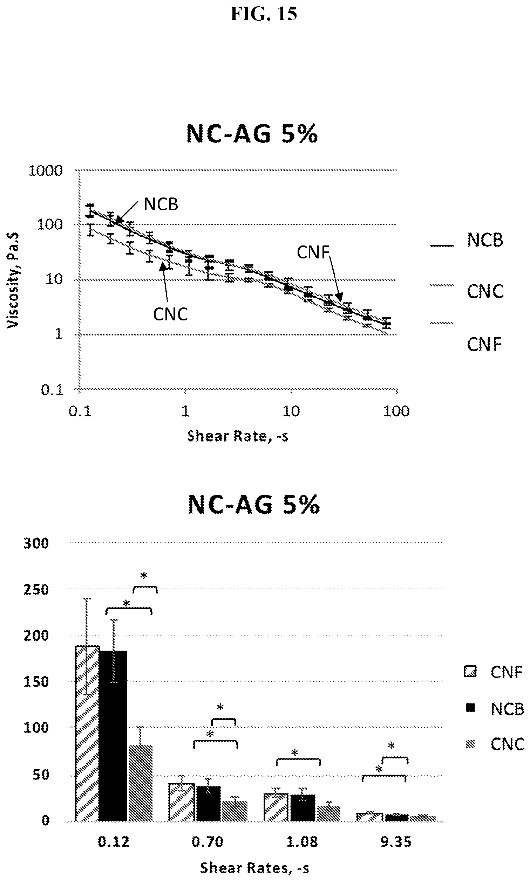

Shear rate ramps were performed to investigate the flow properties of CNF-AG, CNC-AG and NCB-AG bioinks by measuring the viscosity as a function of increasing shear rate (FIGS. 13-15). FIGS. 13 to 15 show the viscosity for different nanocellulose-alginate bioinks as a function of shear rate, for CNC, NCB, and CNF bioinks combined with 1.25%, 2.5%, and 5% alginate (w/v), respectively. Data is expressed as the mean ± SD (*n* = 3 for all groups). Statistical differences are calculated using t-tests, and * denotes *p* < 0.05.

All nanocellulose-containing bioinks exhibited a higher degree of non-Newtonian, shear-thinning, pseudoelastic behavior compared to pure alginate bioinks. CNF-AG and NCB-AG formulations demonstrated greater and statistically different viscosities compared to all CNC formulations. This difference is best seen at shear rate = 0.12 s⁻¹ but is still present at higher shear rates (FIGS. 13-15, bottom panels). Without being limited by speculation, this result implies that there are stronger particle-particle interactions or increased entanglements between nanostructures and microstructures within the NCB-AG and CNF-AG, compared to CNC-AG bioinks, providing better shape fidelity post-printing.

### BioPlus^{®} Nanocellulose is Biocompatible with Human Nasoseptal Chondrocytes

FIG. 16 shows viability of human nasoseptal chondrocytes after bioprinting, for (A) nanocellulose crystal (CNC-AG), (B) nanocellulose blend (NCB-AG), and (C) nanocellulose fibril (CNF-AG) bioink formulations. Data is expressed as the mean ± SD (*n* = 5 for all groups). Statistical differences are calculated by one-way ANOVA with Tukey's HSD multiple comparison post-hoc test (*p* =0.1502, n = 5 with 3 biological repeats).

According to FIG. 16, immediately following bioprinting, cell viability was highest for NCB-AG (83.9 ± 16.7% live cells), followed by CNC-AG (80.1 ± 17.4% live cells) and lastly CNF-AG (71.6 ± 17.4% live cells) but differences were not statistically significant (*p* = 0.15).

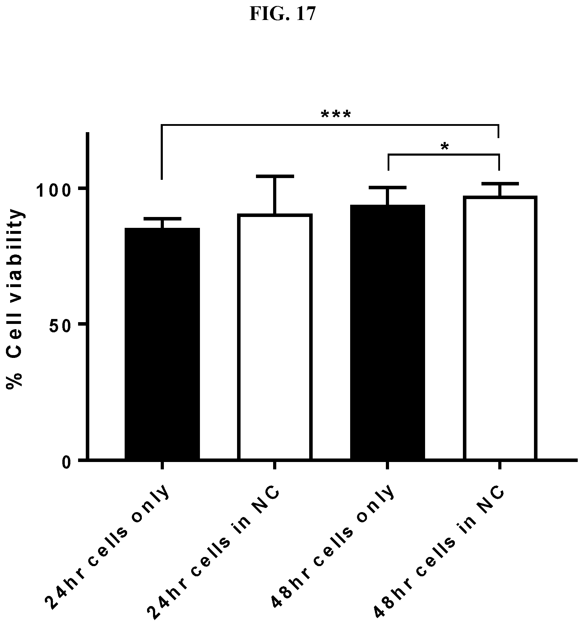

FIG. 17 shows human nasoseptal chondrocyte viability in crystal/fibril blended nanocellulose bioink, in 3D cell culture, versus cells only in 2D cell culture. White bars indicate viability post-bioprinting with NCB-AG after 24 and 48 hours in 3D culture, while black bars after 24 and 48 hours indicate viability post-bioprinting with NCB-AG after 24 and 48 hours in 2D culture. Data is expressed as the mean ± SD *(n* = 4 for all groups). Statistical differences are calculated by the Mann-Whitney test. In FIG. 17, * denotes *p* < 0.05, and *** denotes *p* < 0.001.

Constructs bioprinted using NCB-AG and crosslinked with 0.5 M CaCl₂ demonstrated the greatest volume stability and favorable cell viability. This formulation was therefore used for further biocompatibility experiments. Cell viability in NCB-AG (90.1 ± 13.8%) after 24 hours in culture was comparable to cells on tissue culture plastic alone (85.3 ± 3.4%). However, surprisingly, by 48 hours, cell viability was significantly higher for cells cultured in NCB-AG (97.3 ± 4.5%) compared to cells cultured on plastic alone (94.0% ± 6.4%, *p* = 0.0326) (FIG. 17).

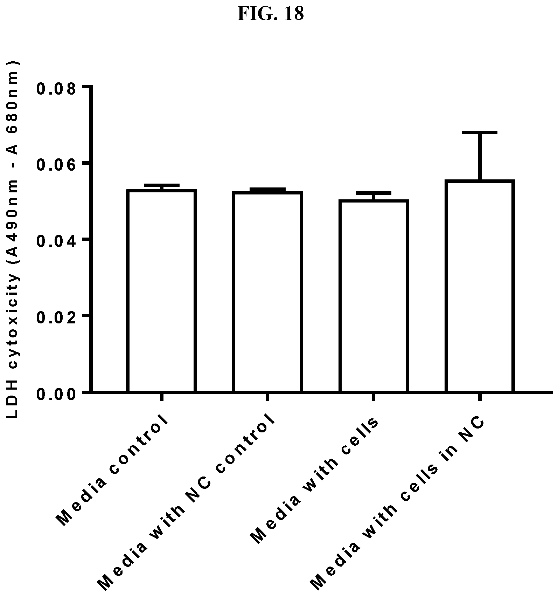

FIG. 18 summarizes the results of a lactate dehydrogenase (LDH) cytotoxicity assay. Cytotoxicity of human nasoseptal chondrocytes in 2D culture is compared to cytotoxicity of human nasoseptal chondrocytes post-bioprinting with NCB-AG bioink. Data is expressed as the mean ± SD. Statistical differences between media with cells and NCB-AG bioink was calculated by the unpaired t-test (*p* = 0.322, n = 3-6). Median LDH activity, an indicator of cytotoxicity, was not found to be significantly different (*p* = 0.1138) between cells cultured on plastic (LDH activity = 0.05) versus cells that were bioprinted and cultured in 3D in NCB-AG (LDH activity = 0.049).

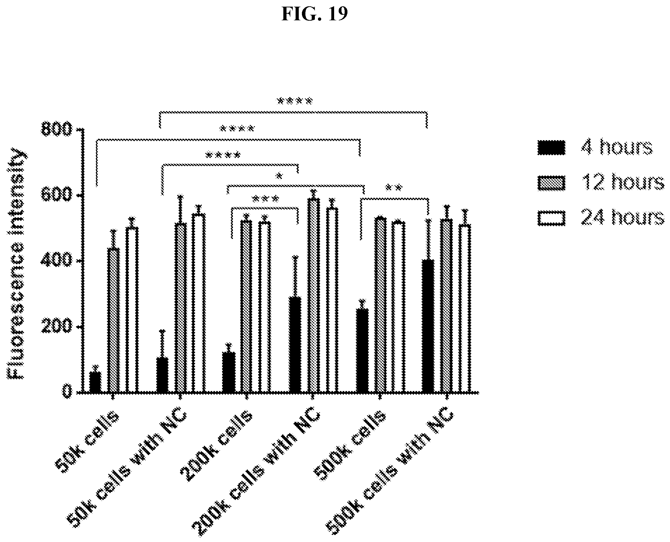

FIG. 19 shows metabolic activity of human nasoseptal chondrocytes in NCB-AG bioink at 4, 12, and 24 hours. Metabolic activity is indicated by the fluorescence signal caused by reduction of AlamarBlue (with the medium or medium plus nanocellulose value subtracted) in wells with increasing numbers of cells. Data is expressed as the mean ± SD (*n =* 5 for all groups). Statistical differences are calculated by one-way ANOVA with Tukey HSD multiple comparison post-hoc test. In FIG. 19, * denotes *p* < 0.05, ** denotes *p* < 0.01, *** denotes *p* < 0.001, and **** denotes *p* < 0.0001.

Human nasoseptal chondrocytes had significantly increased metabolic activity between 4 and 12 hours in culture at all cell densities and conditions tested (*p* < 0.0001) but not between 12 and 24 hours (FIG. 19). Chondrocytes demonstrated significantly increased metabolic activity in NCB-AG bioink compared to culture in 2D (cells only) on plastic after four hours, as measured by the fluorescence intensity for 2 × 10⁵ cells (122.2 ± 23.9 vs. 293.0 ± 118.1 respectively; *p* = 0.0005) and 5 × 10⁵ (257.8 ± 21.0 vs 403.0 ± 120.5 respectively; *p* = 0.0068) cells (FIG. 19). At 12 and 24 hours, the differences between the conditions are no longer significant.

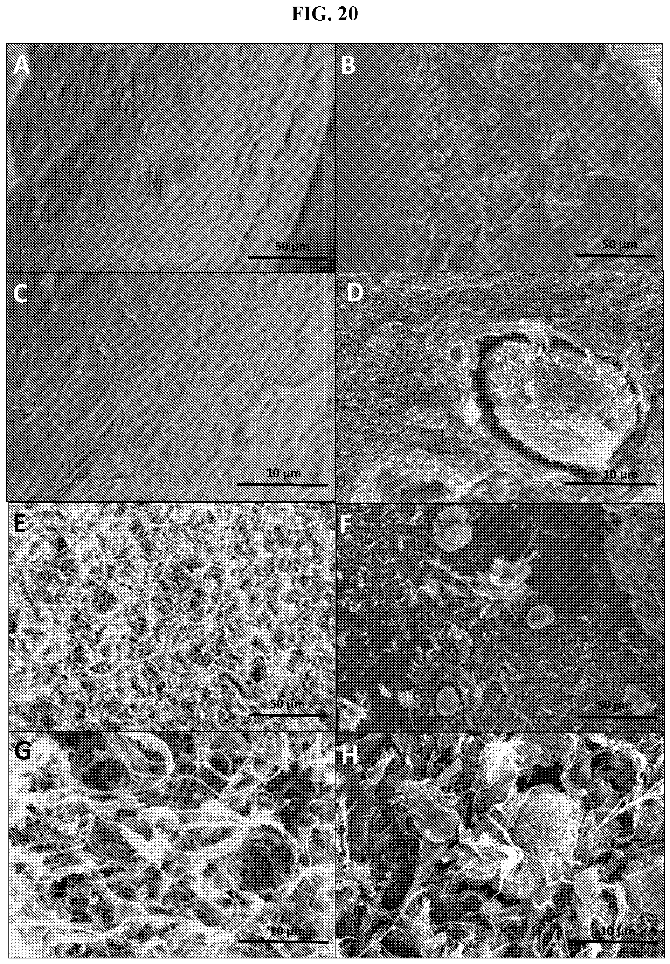

FIG. 20 shows scanning electron microscopy (SEM) images of alginate bioink and nanocellulose-containing bioink constructs following bioprinting. Panels A and C show alginate bioinks without cells. Panels B and D show alginate bioinks after 21 days in culture with human nasoseptal chondrocytes. Panels E and G show NCB-AG bioinks without cells. Panels F and H show NCB-AG bioinks after 21 days in culture with human nasoseptal chondrocytes. The scale bar of the SEM images in panels A, B, E, and H is 50 µm, and the scale bar of the SEM images in panels C, D, G, and H is 10 µm.

SEM images demonstrate the relatively homogenous nature of 2.5% (w/v) alginate bioink (panels A and C) compared to the highly porous structure of the biostructure derived from nanocellulose-blend bioink (panels E and G). The varied nanoarchitecture and microarchitecture of the nanocellulose-containing biostructure provides a larger surface area for cell adhesion. Human nasoseptal chondrocytes maintain a rounded cell morphology in both pure alginate (panels B and D) and nanocellulose blend (panels F and H) after 3 weeks in culture.

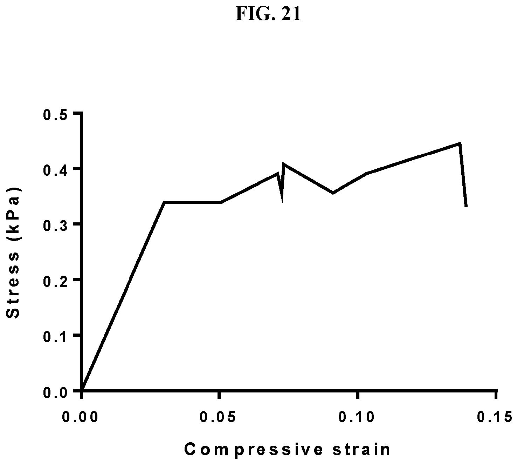

FIG. 21 is a stress-strain curve for bioprinted constructs. Unconfined compression testing of bioprinted NCB-AG constructs crosslinked with 0.5 M CaCl₂ demonstrated a compressive/Young's Modulus of 52.6 kPa.

### Chondrogenesis Results for BioPlus^{®} Nanocellulose-Based Bioinks

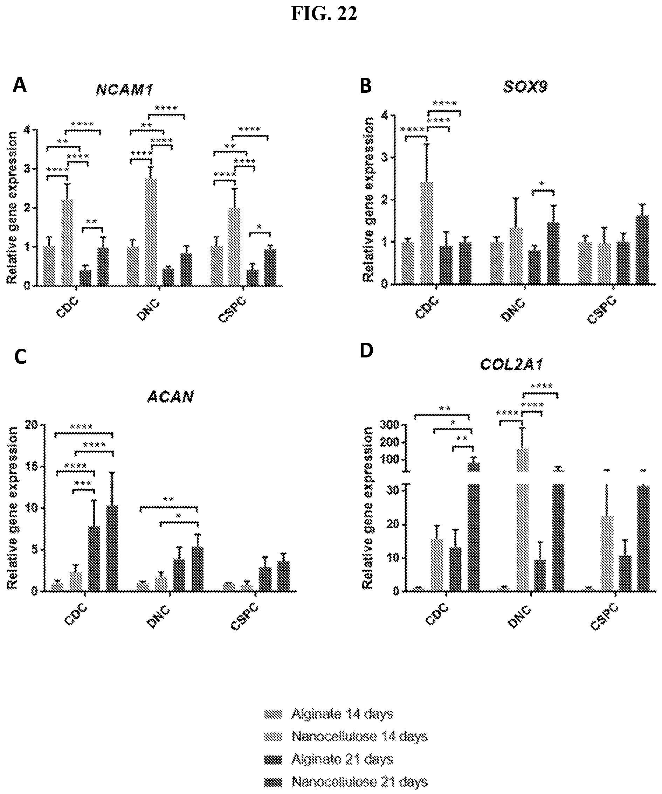

FIG. 22 shows quantitative reverse transcription polymerase chain reaction (qRT-PCR) analysis of relative gene expression of nasoseptal populations cultured in alginate versus nanocellulose-containing bioinks. CDC, cartilage derived cells; DNC, differentiated chondrocytes; and CSPC, cartilage derived stem/progenitor cells were bioprinted using pure alginate and nanocellulose-alginate bioinks (using a nanocellulose crystal/fibril blend). Pellets were cultured for 14 and 21 days and analyzed for expression of *NCAM1,* neural cell adhesion molecule 1 (panel A); *SOX9,* SRY-Box 9 (panel B), *ACAN,* Aggrecan (panel C); and *COL2A1,* collagen type II (panel D). In each panel, and for each grouping of 4 bars, from left to right the bars are for alginate at 14 days (medium-light gray), nanocellulose-containing bioink at 14 days (light gray), alginate at 21 days (medium-dark gray), and nanocellulose-containing bioink at 21 days (dark gray).

The data shown is the ratio of the concentration of the gene of interest to the geometric mean of ribosomal protein L3 (*RPL3*) and TATA-box binding protein (*TBP*) housekeeping genes. Data is expressed as the mean ± SD (*n =* 5), where the alginate at 14 days group for each cell type, was used as the calibrator group (normalized to expression level 1). Statistical differences are calculated by one-way ANOVA with Tukey HSD multiple comparison post-hoc test. In FIG. 22, * denotes *p* < 0.05, ** denotes *p* < 0.01, *** denotes *P* < 0.001, and **** denotes *p <* 0.0001.

Gene expression analysis was undertaken on constructs bioprinted using pure alginate and nanocellulose bioinks with CDC, DNC and CSPC populations, after 14 and 21 days in culture (FIG. 22). Data shows that *NCAM1* expression peaks after 14 days in culture for all three cell types in both bioinks, but it is significantly increased in nanocellulose compared to alginate constructs (panel A of FIG. 22). The early chondrogenesis gene *SOX9* has significantly greater relative expression in nanocellulose-CDC compared to alginate-CDC constructs after 14 days in culture (1.0 ± 0.09 versus 2.3 ± 0.89; *p* < 0.0001) and this reduces 2.3-fold by 21 days (*p* < 0.0001) to reach similar levels to alginate-CDC constructs (panel B of FIG. 22). This S*OX9* expression peak at 14 days does not occur in DNC and CSPC populations. *ACAN* on the other hand demonstrates a consistent pattern of increasing expression with increasing time in culture, with significant differences in the DNC-nanocellulose constructs (3.0-fold, *p* = 0.0355), CDC-alginate (7.6-fold, *p <* 0.0001), and CDC-nanocellulose constructs (4.6-fold, *p* < 0.0001) at 14 and 21 days, respectively (panel C, FIG. 22).

Although nanocellulose constructs appear to have a pattern of increased expression compared to alginate constructs, these differences were not shown to be significant at matched time points. *COL2A1* expression on the other hand showed 6.4-fold greater expression in CDC-nanocellulose compared to CDC-alginate constructs after 21 days (*p* = 0.006) (panel D of FIG. 22), indicating potential for chondrogenicity if these mRNA levels are translated into extracellular matrix secretion. Although DNC-nanocellulose constructs demonstrated a significant dramatic peak in *COL2A1* expression at 14 days compared to DNC-alginate constructs (*p <* 0.0001), this was not sustained by 21 days (*p =* 0.54).

This quantitative reverse transcription polymerase chain reaction data indicates that CDC-nanocellulose constructs have the most promising chondrogenic gene expression profiles for cartilage formation.

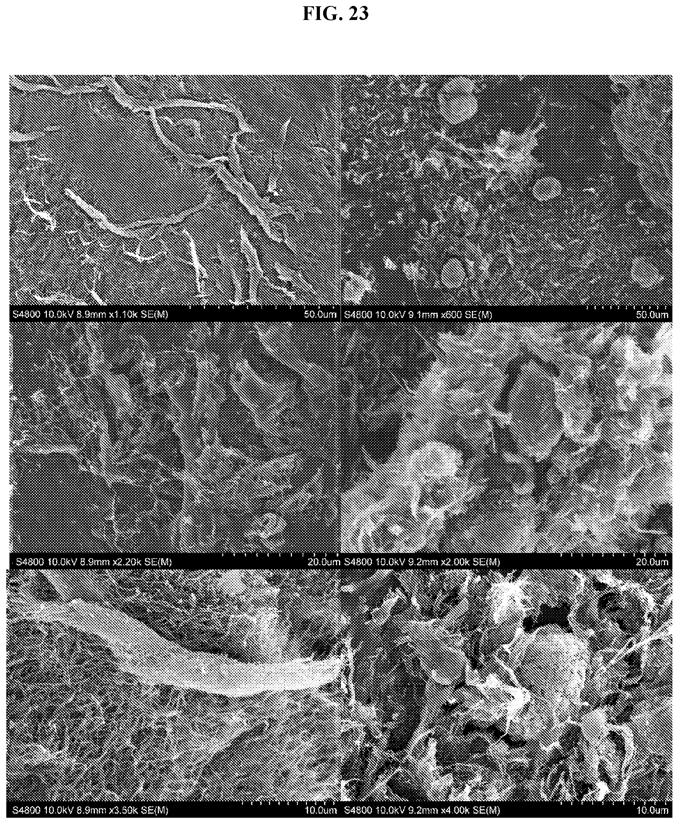

Human nasoseptal chondrocytes were bioprinted using pure alginate and nanocellulose bioinks. SEM analysis was undertaken on samples immediately post-bioprinting with nanocellulose demonstrating nasoseptal chondrocytes with elongated, fibroblast-like appearance. FIG. 23 shows SEM images (scale bars of 10, 20, and 50 µm from bottom to top) of nanocellulose-containing bioink within 24 hours of being mixed with nasoseptal chondrocytes (left panels) and after two weeks in culture with nasoseptal chondrocytes (right panels). FIG. 23 demonstrates the cell change from a fibroblastic to rounded morphology, indicating chondrogenic phenotype. Following three weeks of culture, nasoseptal chondrocytes assume a rounded, chondrogenic phenotype in nanocellulose.

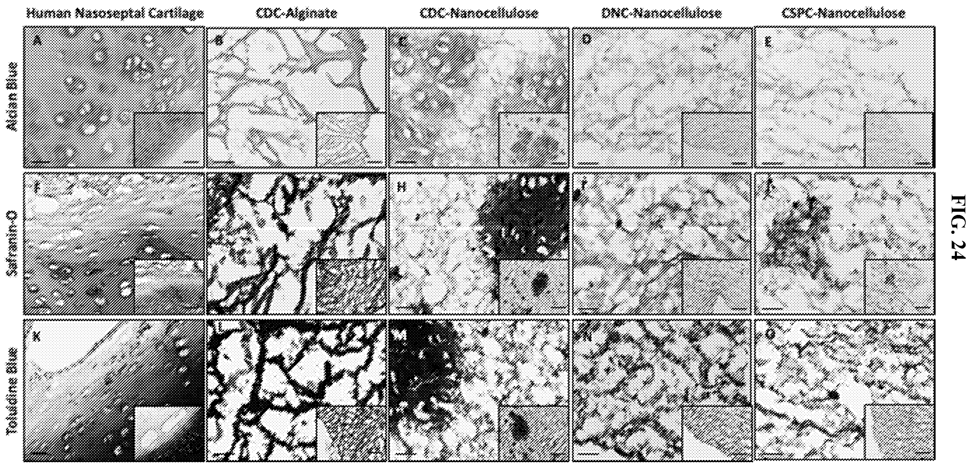

FIG. 24 presents a histological analysis of 3D-bioprinted cartilage constructs using cells derived from nasoseptal cartilage. CDC, cartilage derived cells (panels C, H, and M); DNC, differentiated chondrocytes (panels D, I, and N); CSPC, cartilage derived stem/progenitor cells (panels E, J, and O), bioprinted using nanocellulose-alginate bioinks are compared to CDCs bioprinted with pure alginate bioink (panels B, G, and L). Sections were stained with alcian blue (panels A, B, C, D, and E), safranin-O (panels F, G, H, I, and J) and toluidine blue (panels K, L, M, N, and O) after 21 days in culture. Native human nasoseptal cartilage sections were stained using matching protocols (panels A, F, and K). The scale bar is 20 µL for all panels of FIG. 24.

Histology confirmed that CDC-nanocellulose constructs demonstrated the most cartilage extracellular matrix. Glycosaminoglycans in constructs bioprinted with pure alginate versus nanocellulose bioinks were compared to native human nasoseptal cartilage sections stained using matching protocols (FIG. 24). No visible extracellular matrix morphology was evident on sections from CDC-alginate (panels B, G, and L) and DNC-nanocellulose constructs (panels D, I, and N). Alcian Blue (panels A-E) and Toluidine Blue (panels K-O) demonstrate sulfated proteoglycans throughout human nasoseptal cartilage (panel A) and clusters of sulfated proteoglycans in CDC-nanocellulose constructs, particularly in the pericellular matrix regions surrounding identifiable lacunae (panels C and M). Safranin-O staining also shows nodules intense deep red positivity in CDC-nanocellulose constructs, indicating increased glycosaminoglycan content in areas of new extracellular cartilage formation (panel H). There was also evidence of smaller nodules containing glycosaminoglycans in CSPC-nanocellulose constructs with less intense Safranin-O (panel J) staining but no Alcian Blue or Toluidine Blue positivity (panels E and O, respectively).

Given collagen type II and aggrecan are the major components of the extracellular matrix of human nasoseptal hyaline cartilage, sustained expression of these genes by chondrocytes cultured in nanocellulose suggests this material is providing the right environmental cues for cartilage formation. The transcription data is supported with increased glycosaminoglycan histological staining, providing evidence of extracellular matrix secretion as well as morphological organization of cartilage nodules with lacunae formation with nanocellulose. These findings were absent in alginate constructs, despite the rounded phenotype seen on SEM after three weeks-indicating that although 3D cell culture maintains rounded phenotype, this alone is not sufficient (in alginate constructs) for chondrocyte differentiation and extracellular matrix secretion.

The results of these Examples demonstrate that optimized crystal, fibril, and blend nanocellulose-alginate bioink formulations have comparable resolution, suitable rheology, and biocompatibility with human nasoseptal chondrocytes to enable utility for cartilage bioprinting.

The nanocellulose-alginate bioink with both nanocellulose fibrils and crystals offers the advantage of exhibiting nanoscale roughness as well as microscale roughness for cellular functionality, as well as maintaining a stable construct volume, to reliably maintain the shape of patient-specific cartilage constructs under culture conditions.

TEM and SEM data shows that even with addition of alginate, all tested nanocellulose-containing formulations continue to be highly porous both in the nanoscale and microscale, unlike pure alginate bioinks which are typically only nanoporous (at length scales of about 5 nm). NCB hydrogels exhibited a combination of fibrillar networks and interconnecting compact nanorods and have a broader variation in pore sizes (55 nm to over 12 µm, with mean of 934 nm) compared to those from CNC alone, which would allow chondrocyte migration (approximate chondrocyte diameter is 10 µm) and provide a tunable nanotopography for a greater variety of bioactive signals.

The addition of alginate to allow crosslinking capability did not adversely affect the elasticity or shear-thinning behavior of nanocellulose bioinks, which is believed to depend primarily on the dry content of the bioink rather than the ratio between the two components. The storage modulus was greater than the loss modulus for all combinations of nanocellulose-alginate bioinks, over all frequencies studied, exhibiting a dominance of elasticity similar to pure nanocellulose materials. This was in contrast to pure alginate bioinks, which are predominantly viscous. The large aspect ratio and ability to form interconnected network structures through hydrogen bonding makes nanocellulose both stiff due to the ordered (crystalline) regions and flexible due to the disordered (amorphous) regions of the nanoparticles. These nanocellulose network structures can disentangle and align parallel to the direction of flow when placed in suspension, which also accounts for the high degree of shear thinning observed. Shear thinning enables bioprinting through fine deposition nozzles at low shear rates with reduced mechanical forces exerted on cells. This is in contrast to the more commonly used pure-alginate bioinks, which exhibit only limited shear thinning even at high shear rates requiring higher forces (shear stress) for extrusion which can reduce post-printing cell viability. Reversible stress-softening behavior of nanocellulose means that post-printing, the storage modulus (G') is recovered under static conditions and is higher than that for pure alginate bioinks (in the 1.25% to 7.5% w/v range) contributing to shape fidelity and preventing collapse of complex 3D-bioprinted constructs. The rigidity of NCB was significantly higher than for CNC but similar to CNF, indicating that the double network hydrogel structure previously reported (Kyle et al., "Characterization of pulp derived nanocellulose hydrogels using AVAP® technology", Carbohydrate Polymers 198 (2018) 270-280) for NCB is not disrupted by the addition of alginate.

Nanocellulose is broadly considered to be biocompatible using *in vivo* animal studies of bacterial nanocellulose (BNC) demonstrating no foreign body reaction or inflammation. Although it is well-known that cellulose is not readily degraded by the human body due to the lack cellulolytic enzymes, spontaneous non-enzymatic hydrolysis of cellulose chains has been suggested to account for slow breakdown which can be enhanced through oxidation. Hydrolysis of nanocellulose results in glucose, which is not toxic. Even if not fully broken down, cellulose is biologically inert and theoretically should not interfere with homeostatic processes such as matrix remodeling. Most reports on the nanotoxicology of nanocellulose use BNC and show no evidence of damage at the genetic, cellular, and *in vivo* animal level, but few studies use human cells and none heretofore directly compare pulp-based crystal, fibril, and blend formulations.

We find that BioPlus^{®} pulp-based nanocellulose is non-cytotoxic to nanoseptal chondrocytes with no significant differences in cell viability post-printing with CNC, NCB and CNF. NCB not only significantly increased the proportion of viable nasoseptal chondrocytes in culture after two days, it also encouraged them to be more metabolically active after the first four hours post printing, while maintaining a rounded chondrogenic phenotype after three weeks in culture-suggesting a suitable 3D environment, nanotopography, and porosity for cartilage matrix formation. These findings are consistent with previous studies indicating that maintenance of a round cell morphology is a prerequisite for overt chondrogenic differentiation. The nanocellular fibrillary structures can mimic the properties of extracellular matrix components, which are also nanofibrillar networks composed of glycosaminoglycans and fibrous proteins, such as collagen, elastin, laminin, and fibronectin, thereby encouraging cells to differentiate.

Chondrogenesis results were positive for the nanocellulose-based bioinks. The presence of nanocellulose encourages a cell change from a fibroblastic to rounded morphology, indicating chondrogenic phenotype. It has been experimentally demonstrated that the disclosed nanocellulose-containing bioinks promote cartilage formation.

## Claims

1. A bioink composition for 3D bioprinting, said bioink composition comprising:
(a) nanocellulose in the form of a combination of nanocellulose crystals and nanocellulose fibrils;
(b) alginate in the form of alginic acid and/or an alginate salt; and
(c) water;
wherein said alginate is ionically crosslinkable in the presence of an ionic crosslinking agent.

2. The bioink composition of claim 1 , wherein said nanocellulose is present at a nanocellulose concentration from 1% (w/v) to 10% (w/v), for example, wherein said nanocellulose is present at a nanocellulose concentration from 3% (w/v) to 6% (w/v), for example, wherein said alginate is present at an alginate concentration from 0.1% (w/v) to 5% (w/v).

3. The bioink composition of any of claims 1 to 2, wherein said bioink composition is a cell-free bioink.

4. The bioink composition of any of claims 1 to 2, wherein said bioink composition is a cell-laden bioink containing viable human cells, and wherein said viable human cells are optionally human nasoseptal chondrocytes, for example, wherein said viable human cells are present in said bioink composition at a cell concentration from 10⁵ to 10⁷ cells per milliliter of said bioink composition.

5. The bioink composition of any of claims 1 to 4, wherein said nanocellulose is hydrophilic nanocellulose.

6. The bioink composition of claim 1, wherein said nanocellulose is **characterized by** a thermal-decomposition onset temperature of at least 250°C, for example, wherein said thermal-decomposition onset temperature is at least 290°C.

7. The bioink composition of any of claims 1 to 6, wherein said nanocellulose contains less than 0.05 wt% sulfur, for example, wherein said nanocellulose contains no sulfate half-ester groups.

8. The bioink composition of any of claims 1 to 7, wherein said nanocellulose has a zeta potential of at least -20 mV or more negative.

9. The bioink composition of any of claims 1 to 8, wherein said nanocellulose is shear-thinning.

10. The bioink composition of any of claims 1 to 9, wherein said nanocellulose is derived from fractionation of lignocellulosic biomass in the presence of sulfur dioxide and ethanol to generate cellulose, hemicellulose, and lignin, followed by mechanical refining of said cellulose to generate said nanocellulose.

11. The bioink composition of any of claims 1 to 10 wherein said nanocellulose is not bacterial nanocellulose, wherein said nanocellulose is not derived from tunicates, and wherein said nanocellulose is not obtained via enzymatic hydrolysis of lignocellulosic biomass or cellulose.

12. The bioink composition of any of claims 1 to 11, wherein said bioink composition further comprises an ionic crosslinking agent, for example, wherein said ionic crosslinking agent includes a divalent cation, for example, wherein said divalent cation is selected from the group consisting of Ca²⁺, Sr²⁺, Ba²⁺, and combinations thereof.

13. The bioink composition of any of claims 1 to 12, said bioink composition further comprising one or more bioink additives selected from the group consisting of growth promoters, growth factors, vitamins, minerals, enzymes, proteins, sugars, sugar alcohols, acids, bases, salts, buffers, stabilizers, dissolved oxygen, and antibiotics.

14. The bioink composition of any of claims 1 to 13, wherein said bioink composition is non-cytotoxic to human cells.

## Patentansprüche

1. Biotintenzusammensetzung zum 3D-Biodruck, wobei die Biotintenzusammensetzung umfasst:
(a) Nanocellulose in der Form einer Kombination von Nanocellulosekristallen und Nanocellulosefibrillen;
(b) Alginat in der Form von Alginsäure und/oder einem Alginatsalz; und
(c) Wasser;
wobei das Alginat in Gegenwart eines ionischen Vernetzungsmittels ionisch vernetzbar ist.

2. Biotintenzusammensetzung nach Anspruch 1, wobei die Nanocellulose in einer Nanocellulose-Konzentration von 1 % (w/v) bis 10 % (w/v) vorhanden ist, wobei die Nanocellulose beispielsweise in einer Nanocellulose-Konzentration von 3 % (w/v) bis 6 % (w/v) vorhanden ist, wobei das Alginat beispielsweise in einer Alginat-Konzentration von 0,1 % (w/v) bis 5 % (w/v) vorhanden ist.

3. Biotintenzusammensetzung nach einem der Ansprüche 1 bis 2, wobei die Biotintenzusammensetzung eine zellfreie Biotinte ist.

4. Biotintenzusammensetzung nach einem der Ansprüche 1 bis 2, wobei die Biotintenzusammensetzung eine zellbeladene Biotinte ist, die lebensfähige menschliche Zellen enthält, und wobei die lebensfähigen menschlichen Zellen gegebenenfalls menschliche Nasoseptum-Chondrozyten sind, wobei die lebensfähigen menschlichen Zellen beispielsweise in der Biotintenzusammensetzung in einer Zellkonzentration von 10⁵ bis 10⁷-Zellen pro Milliliter der Biotintenzusammensetzung vorhanden sind.

5. Biotintenzusammensetzung nach einem der Ansprüche 1 bis 4, wobei die Nanocellulose hydrophile Nanocellulose ist.

6. Biotintenzusammensetzung nach Anspruch 1, wobei die Nanocellulose **gekennzeichnet ist durch** eine Temperatur des Einsetzens von thermischer Zersetzung von wenigstens 250 °C, wobei die Temperatur des Einsetzens von thermischer Zersetzung beispielsweise wenigstens 290 °C beträgt.

7. Biotintenzusammensetzung nach einem der Ansprüche 1 bis 6, wobei die Nanocellulose weniger als 0,05 Gew.-% Schwefel enthält, wobei die Nanocellulose beispielsweise keine Sulfathalbestergruppen enthält.

8. Biotintenzusammensetzung nach einem der Ansprüche 1 bis 7, wobei die Nanocellulose ein Zetapotential von wenigstens -20 mV oder stärker negativ aufweist.

9. Biotintenzusammensetzung nach einem der Ansprüche 1 bis 8, wobei die Nanocellulose scherverdünnend ist.

10. Biotintenzusammensetzung nach einem der Ansprüche 1 bis 9, wobei die Nanocellulose durch Fraktionierung von lignocellulosehaltiger Biomasse in Gegenwart von Schwefeldioxid und Ethanol, um Cellulose, Hemicellulose und Lignin zu erzeugen, gefolgt von mechanischer Raffination der Cellulose, um die Nanocellulose zu erzeugen, abgeleitet ist.

11. Biotintenzusammensetzung nach einem der Ansprüche 1 bis 10, wobei die Nanocellulose keine bakterielle Nanocellulose ist, wobei die Nanocellulose nicht von Tunicaten abgeleitet ist und wobei die Nanocellulose nicht durch enzymatische Hydrolyse von lignocellulosehaltiger Biomasse oder Cellulose erhalten ist.

12. Biotintenzusammensetzung nach einem der Ansprüche 1 bis 11, wobei die Biotintenzusammensetzung ferner ein ionisches Vernetzungsmittel umfasst, wobei das ionische Vernetzungsmittel beispielsweise ein zweiwertiges Kation umfasst, wobei das zweiwertige Kation ausgewählt ist aus der Gruppe bestehend aus Ca²⁺, Sr²⁺, Ba²⁺ und Kombinationen davon.

13. Biotintenzusammensetzung nach einem der Ansprüche 1 bis 12, wobei die Biotintenzusammensetzung ferner einen oder mehrere Biotinten-Zusatzstoffe ausgewählt aus der Gruppe bestehend aus Wachstumsförderern, Wachstumsfaktoren, Vitaminen, Mineralstoffen, Enzymen, Proteinen, Zuckern, Zuckeralkoholen, Säuren, Basen, Salzen, Puffern, Stabilisatoren, gelöstem Sauerstoff und Antibiotika umfasst.

14. Biotintenzusammensetzung nach einem der Ansprüche 1 bis 13, wobei die Biotintenzusammensetzung für menschliche Zellen nicht zytotoxisch ist.

## Revendications

1. Composition de bioencre pour bio-impression 3D, ladite composition de bioencre comprenant :
(a) une nanocellulose sous forme d'une combinaison de cristaux de nanocellulose et de fibrilles de nanocellulose ;
(b) un alginate sous forme d'acide alginique et/ou un sel d'alginate ; et
(c) de l'eau ;
dans laquelle ledit alginate est réticulable ioniquement en présence d'un agent de réticulation ionique.

2. Composition de bioencre selon la revendication 1, dans laquelle ladite nanocellulose est présente à une concentration de nanocellulose de 1 % (p/v) à 10 % (p/v), par exemple, dans laquelle ladite nanocellulose est présente à une concentration de nanocellulose de 3 % (p/v) à 6 % (p/v), par exemple, dans laquelle ledit alginate est présent à une concentration d'alginate de 0,1 % (p/v) à 5 % (p/v).

3. Composition de bioencre selon l'une quelconque des revendications 1 et 2, dans laquelle ladite composition de bioencre est une bioencre exempte de cellules.

4. Composition de bioencre selon l'une quelconque des revendications 1 et 2, dans laquelle ladite composition de bioencre est une bioencre chargée en cellules contenant des cellules humaines viables, et dans laquelle lesdites cellules humaines viables sont éventuellement des chondrocytes nasoseptaux humains, par exemple, dans laquelle lesdites cellules humaines viables sont présentes dans ladite composition de bioencre à une concentration en cellules de 10⁵ à 10⁷ cellules par millilitre de ladite composition de bioencre.

5. Composition de bioencre selon l'une quelconque des revendications 1 à 4, dans laquelle ladite nanocellulose est une nanocellulose hydrophile.

6. Composition de bioencre selon la revendication 1, dans laquelle ladite nanocellulose est **caractérisée par** une température de début de décomposition thermique d'au moins 250 °C, par exemple, dans laquelle ladite température de début de décomposition thermique est d'au moins 290 °C.

7. Composition de bioencre selon l'une quelconque des revendications 1 à 6, dans laquelle ladite nanocellulose contient moins de 0,05 % en poids de soufre, par exemple, dans laquelle ladite nanocellulose ne contient pas de groupes demi-ester de sulfate.

8. Composition de bioencre selon l'une quelconque des revendications 1 à 7, dans laquelle ladite nanocellulose a un potentiel zêta d'au moins -20 mV ou plus négatif.

9. Composition de bioencre selon l'une quelconque des revendications 1 à 8, dans laquelle ladite nanocellulose est fluidifiante par cisaillement.

10. Composition de bioencre selon l'une quelconque des revendications 1 à 9, dans laquelle ladite nanocellulose est dérivée du fractionnement de biomasse lignocellulosique en présence de dioxyde de soufre et d'éthanol pour générer de la cellulose, de l'hémicellulose et de la lignine, suivi par un raffinage mécanique de ladite cellulose pour générer ladite nanocellulose.

11. Composition de bioencre selon l'une quelconque des revendications 1 à 10, dans laquelle ladite nanocellulose n'est pas une nanocellulose bactérienne, dans laquelle ladite nanocellulose n'est pas dérivée de tuniciers, et dans laquelle ladite nanocellulose n'est pas obtenue par hydrolyse enzymatique de biomasse lignocellulosique ou de cellulose.

12. Composition de bioencre selon l'une quelconque des revendications 1 à 11, dans laquelle ladite composition de bioencre comprend en outre un agent de réticulation ionique, par exemple, dans laquelle ledit agent de réticulation ionique comprend un cation divalent, par exemple, dans lequel ledit cation divalent est choisi dans le groupe constitué par Ca²⁺, Sr²⁺, Ba²⁺, et leurs combinaisons.

13. Composition de bioencre selon l'une quelconque des revendications 1 à 12, ladite composition de bioencre comprenant en outre un ou plusieurs additifs de bioencre choisis dans le groupe constitué par les promoteurs de croissance, les facteurs de croissance, les vitamines, les minéraux, les enzymes, les protéines, les sucres, les alcools de sucre, les acides, les bases, les sels, les tampons, des stabilisants, de l'oxygène dissous et des antibiotiques.

14. Composition de bioencre selon l'une quelconque des revendications 1 à 13, dans laquelle ladite composition de bioencre est non cytotoxique pour des cellules humaines.
